# EUROPEAN PATENT SPECIFICATION

(11) **EP 1 169 054 B1**
(45) Date of publication and mention of the grant of the patent: **04.08.2004**
(21) Application number: 00921845.4
(22) Date of filing: 06.04.2000
(51) Int. Cl.: A61K 38/46, A61K 48/00, C12Q 1/68, G01N 33/573, G01N 33/50, A61K 31/404, A61K 31/517, A61K 31/498, A61K 31/00, C12N 15/55

(54) **PROGNOSIS OF PTP LAR RELATED DISEASES**
PROGNOSE VON PTP LAR VERMITTELTEN KRANKHEITEN
PRONOSTIC DE MALADIES ASSOCIEES A PTP LAR

(30) Priority: 09.04.1999 US 128673 P
(43) Date of publication of application: 09.01.2002
(73) Proprietor: Max-Planck-Gesellschaft zur Förderung der Wissenschaften e.V., 82152 Martinsried (DE)
(72) Inventor: ULLRICH, Axel, Portola Valley, CA 94028 (US); MULLER, Thomas Sugen, Inc., S. San Francisco, CA 94080 (US)
(74) Representative: Viering, Jentschura & Partner
(86) International application number: PCT/US2000/009274
(87) International publication number: WO 2000/061180

(56) References cited:
- WO-A-96/40629
- WO-A-98/38984
- WO-A-99/46237
- ZHAI YF ET AL: "LAR-PTPase cDNA transfection suppression of tumor growth of neu oncogene-transformed human breast carcinoma cells." MOLECULAR CARCINOGENESIS, vol. 14, no. 2, 1995, pages 103-110, XP000960983
- LEVEA C M ET AL: "Breast cancer metastasis: A potential role for the LAR protein tyrosine phosphatase." MODERN PATHOLOGY, vol. 12, no. 1, January 1999 (1999-01), page 25A XP000960992 Annual Meeting of the United States and Canadian Academy of Pathology; San Francisco, 20-26 March 1999
- LU J ET AL: "Profile and differential expression of protein tyrosine phosphatases in mouse pancreatic islet tumor cell lines." PANCREAS, vol. 16, no. 4, May 1998 (1998-05), pages 515-520, XP000960982
- KIKUCHI K ET AL: "Protein phosphatases and cancer" CURRENT TOPICS IN BIOCHEMICAL RESEARCH, vol. 1, 1999, pages 75-87, XP000974851
- KYPTA R M ET AL: "Association between a transmembrane protein tyrosine phosphatase and the cadherin-catenin complex." JOURNAL OF CELL BIOLOGY, vol. 134, no. 6, 1996, pages 1519-1529, XP002155378 cited in the application
- SOMMERS C L ET AL: "Alterations in beta-catenin phosphorylation and plakoglobin expression in human breast cancer cells." CANCER RESEARCH, vol. 54, no. 13, 1994, pages 3544-3552, XP002155379 cited in the application
- MULLER T ET AL: "Phosphorylation and free pool of beta-catenin are regulated by tyrosine kinases and tyrosine phosphatases during epithelial cell migration." JOURNAL OF BIOLOGICAL CHEMISTRY, vol. 274, no. 15, 9 April 1999 (1999-04-09), pages 10173-10183, XP002155380

## Description

### FIELD OF THE INVENTION

The present invention relates, *inter alia*, to methods to modulate epithelial cell migration, and to methods useful for the identification of the various products useful for the treatment of the various diseases and disorders.

### BACKGROUND OF THE INVENTION

The following description of the background of the invention is provided to aid in understanding the invention, but is not admitted to be or to describe prior art to the invention.

The cadherins represent a family of transmembrane receptors, which mediate homophillic, Ca²⁺-dependent cell-cell adhesion. In epithelial cells the members of this family, such as the classical E-, N-, and P-cadherins, are primarily found at the adherens junctions of adjacent cells (Yap, *et al*. (1997) *Annu. Rev. Cell Dev. Biol.* **13**, 119-146.). β-catenin and plakoglobin (γ-catenin) associate directly with the highly conserved cytoplasmic domain of classical cadherins in a mutually exclusive manner (Ozawa, *et al*. (1989) *EMBO J.* **8**, 1711-1718; Hinck, *et al*. (1994) *J. Cell Biol*. **125**, 1327-1340).

The cadherin/catenin-complex is linked via α-catenin either directly (Rimm, *et al*. (1995) *Proc. Natl. Acad. Sci. USA* **92**, 8813-8817) or indirectly to the actin filament network via the actin binding proteins α-actinin or vinculin (Knudsen, *et al*. (1995) *J. Cell Biol*. **130**, 67-77; Weiss, *et al*. (1998) *J. Cell Biol*. **141**, 755-64). The association of the cadherin/catenin-complex with the cytoskeleton is essential for tight cell-cell interaction. Nevertheless, cadherin/catenin mediated cell-cell contacts have to be highly dynamic, since particularly during embryonic development or wound healing, adherens junctions have to be rapidly disassembled and reassembled (Marrs, *et al*. (1996) *Int. Rev. Cytol*. **165**, 159-205).

Downregulation of cadherins results in the separation of neighboring cells, a phenomenon that is observed during embryonic development at the epithelial-mesenchymal transition (EMT)¹ of forming mesoderm (Huber, *et al*. (1996) *Curr. Opin. Cell Biol*. **8**, 685-691), as well as in tumor cells allowing their invasion and dissemination throughout the body (Becker, *et al*. (1994) *Cancer Res.* **54**, 3845-3852). During epithelial-mesenchymal transition, cells transiently lose their epithelial features and acquire a fibroblastoid morphology (Thiery, *et al*. (1985) *Annu. Rev. Cell Biol*. **1**, 91-113). The critical importance of an intact cadherin/catenin complex is underscored by the observation that downregulation of any of its components, results in the loss of the tumor suppressive actions of adherens junctions, and correlates with tumor invasion and metastasis (Birchmeier, *et al*. (1995) *Ciba F. Symp*. **189**, 124-141).

Moreover, the integrity of adherens junctions appears to be dynamically regulated by tyrosine phosphorylation. Transfection of a ν-src oncogene (Behrens, *et al*. (1993) *J. Cell Biol.* **120,** 757-766; Hamaguchi, *et al*. (1993) *EMBO J.* **12,** 307-314) or treatment with growth factors (Shibamoto, *et al*. (1994) *Cell Adhes. Commun.* **1,** 295-305; Fujii, *et al*. (1996) *Exp. Cell Res.* **223,** 50-62) causes unstable cell-cell adhesion. Migration of cells and inhibition of protein tyrosine phosphatases (PTP) enhances this destabilizing effect (Volberg, *et al*. (1992) *EMBO J*. **11**, 1733-1742). The model in which reversible tyrosine phosphorylation serves to regulate cadherin-mediated cell-cell adhesion is further supported by the demonstration of cadherin/catenin-complex association with the receptor tyrosine kinases (RTK) EGF receptor and HER2/Neu (Hoschuetzky, *et al*. (1994) *J. Cell Biol.* **127,** 1375-1380; Ochiai, *et al*. (1994) *Biochem. Biophys. Res. Comm.* **205,** 73-78) as well as with the transmembrane PTPs µ, κ, and λ (Brady-Kalnay, *et al*. (1995) *J. Cell Biol*. **130**, 977-986; Fuchs, *et al*. (1996) *J. Biol. Chem*. **271**, 16712-17719; Cheng, *et al*. (1997) *J. Biol. Chem*. **272**, 7264-7277).

β-catenin and plakoglobin are mammalian homologues of the *Drosophila* protein Armadillo, whose function is critical for normal segmental pattern formation during development (Riggleman, *et al*. (1989) *Genes Dev.* **3**, 96-113). The presence of a repeating 42-amino acid sequence motif defines members of the "armadillo family" (Peifer, *et al*. (1994) *Cell* **76**, 789-791).

Data obtained in the *Drosophila* and *Xenopus* systems suggest an additional function for β-catenin, independent of cadherin-mediated cell adhesion. This involves translocation of β-catenin to the nucleus that is preceded by its accumulation in the cytoplasm. Thus, free β-catenin is involved in transcriptional regulation of specific genes that are essential for embryonic development (Miller, *et al*. (1996) *Genes Dev.* **10,** 2527-2539). The signals resulting in a free pool of β-catenin include the binding of Wingless/Wnt to its transmembrane receptor Frizzled, and the inhibition of the serine/threonine kinase Zeste-white 3 (Shaggy) or its vertebrate homologue glycogen synthase kinase 3 (GSK3; Moon, *et al*. (1997) *Trends Genet.* **13,** 256-258).

Further functions for β-catenin and plakoglobin are indicated by their association with the adenomatous polyposis cell (APC) tumor suppressor protein. This protein is thought to serve as a cytoplasmic effector of β-catenin, negatively regulating the accumulation of cytosolic β-catenin in concert with GSK3 (Bullions, *et al*. (1996) *Curr*. *Opin. Oncol*. **10**, 61-7) and axin/conductin (Ikeda, *et al*. (1998) *Embo J.* **17**, 1371-1384; Behrens, *et al*. (1998) *Science* **280,** 596-599; Sakanaka, *et al*. (1998) *Proc*. *Natl. Acad. Sci. USA* **95,** 3020-3023) by inducing ubiquitin-dependent degradation of β-catenin (Aberle, *et al*. (1997) *EMBO J*. **16**, 3797-3804).

### SUMMARY OF THE INVENTION

During epithelial migration β-catenin accumulates in the cytosol in a free, uncomplexed, and tyrosine phosphorylated form. In contrast, in confluent cells, β-catenin maintains epithelial cell integrity as an essential part of the cadherin/catenin tumor suppressor-system. Thus, tyrosine phosphorylation regulates the function of β-catenin as a signaling molecule during epithelial cell migration. PTP LAR is a modulator of epithelial cell migration. Thus, one function of its phosphatase is in the regulation of cell-cell-contacts and epithelial cell integrity. Further, ectopic expression of PTP LAR inhibits tumor formation in nude mice. A dysfunction of PTP LAR may therefore lead to tumor invasion and metastasis.

The patient is a mammal, and preferably a human.

The term "preventing" refers to decreasing the probability that an organism contracts or develops an abnormal condition.

The term "treating" refers to having a therapeutic effect and at least partially alleviating or abrogating an abnormal condition in the organism.

The term "therapeutic effect" refers to the inhibition or inactivation of factors causing or contributing to the abnormal condition. A therapeutic effect relieves to some extent one or more of the symptoms of the abnormal condition. In reference to the treatment of abnormal conditions, a therapeutic effect can refer to one or more of the following: (a) an increase in the proliferation, growth, and/or differentiation of cells; (b) inhibition (*i.e.*, slowing or stopping) of cell death; (c) inhibition of degeneration; (d) relieving to some extent one or more of the symptoms associated with the abnormal condition; and (e) enhancing the function of the affected population of cells. Specifically, a therapeutic effect includes prevention/inhibition of epithelial cell migration. Compounds demonstrating efficacy against abnormal conditions can be identified as described herein.

The term "abnormal condition" refers to a function in the cells or tissues of an organism that deviates from their normal functions in that organism. An abnormal condition can, for example, relate to cell proliferation, cell differentiation, cell survival, or cell migration.

Abnormal cell proliferative conditions include cancers, metastases, fibrotic and mesangial disorders, abnormal angiogenesis and vasculogenesis, wound healing, psoriasis, diabetes mellitus, and inflammation.

Abnormal differentiation conditions include, but are not limited to neurodegenerative disorders, slow wound healing rates, and slow tissue grafting healing rates.

Abnormal cell survival conditions relate to conditions in which programmed cell death (apoptosis) pathways are activated or abrogated.

Abnormal cell migration conditions include, but are not limited to aberrant wound healing, cancer and metastases.

The term "aberrant", in relation to this invention, refers to lower or higher epithelial cell migration compared with normal cells from the same organism or a different organism. Alternatively, it can refer to over- or under-tyrosine phosphorylation of β-catenin, or higher or lower than normal levels of β-catenin pools. These effects can be modulated by PTP LAR, or by other compounds of the invention.

The term "administering" relates to a method of incorporating a substance into cells or tissues of an organism. The abnormal condition can be prevented or treated when the cells or tissues of the organism exist within the organism or outside of the organism. Cells existing outside the organism can be maintained or grown in cell culture dishes. For cells harbored within the organism, many techniques exist in the art to administer substances, including (but not limited to) oral, parenteral, dermal, injection, and aerosol applications. For cells outside of the organism, multiple techniques exist in the art to administer substances, including (but not limited to) cell microinjection techniques, transformation techniques, and carrier techniques.

The patient is preferably a mammal, including but not limited to, mouse, rat, rabbit, guinea pig, or goat, more preferably a monkey or ape, and most preferably a human.

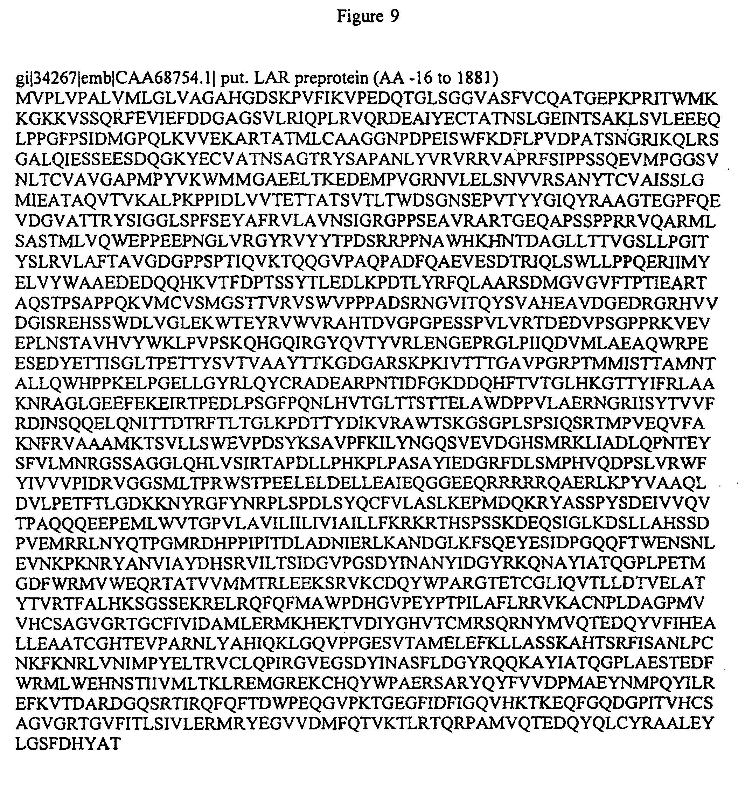

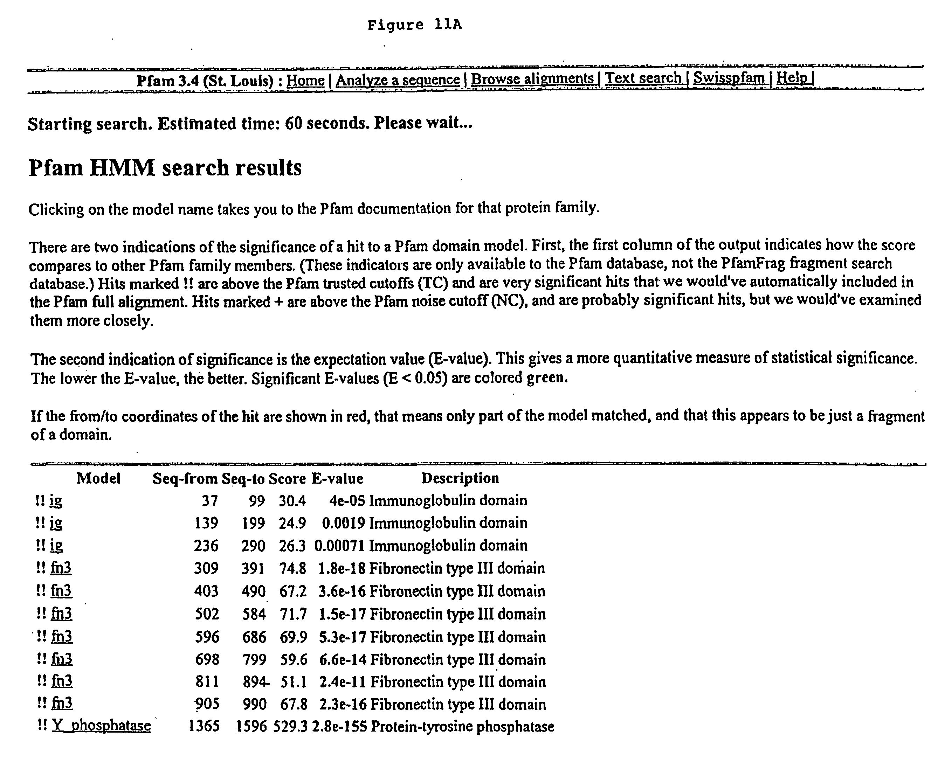

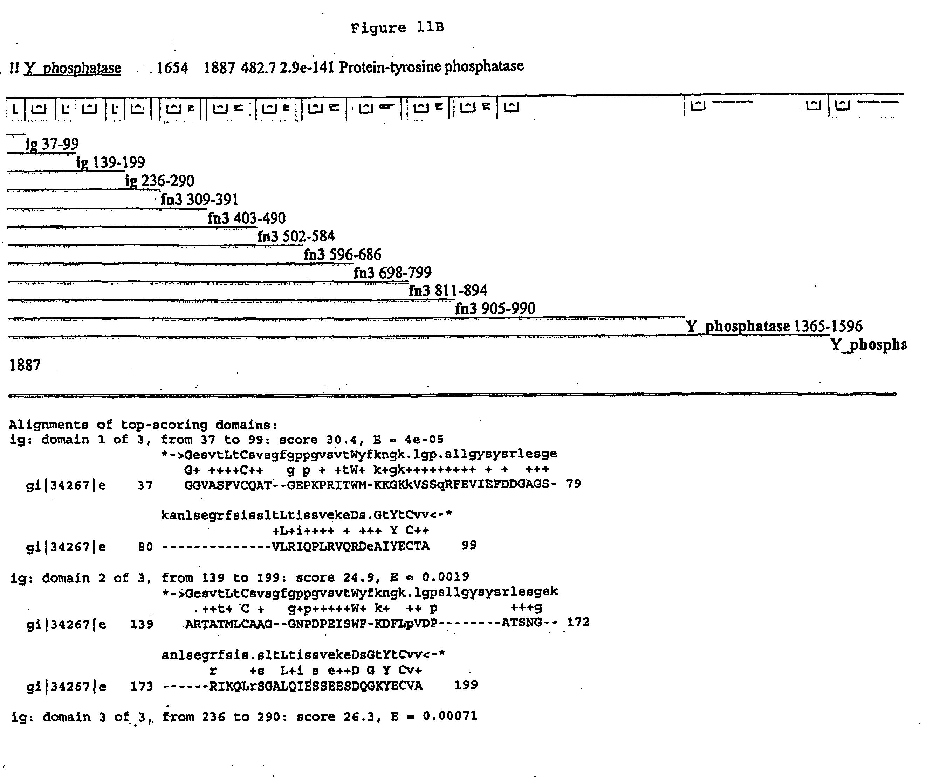

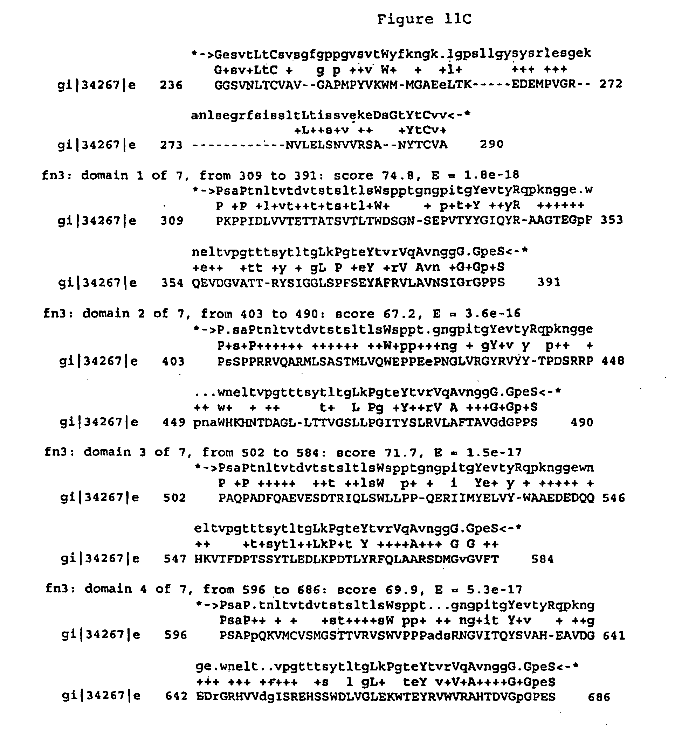

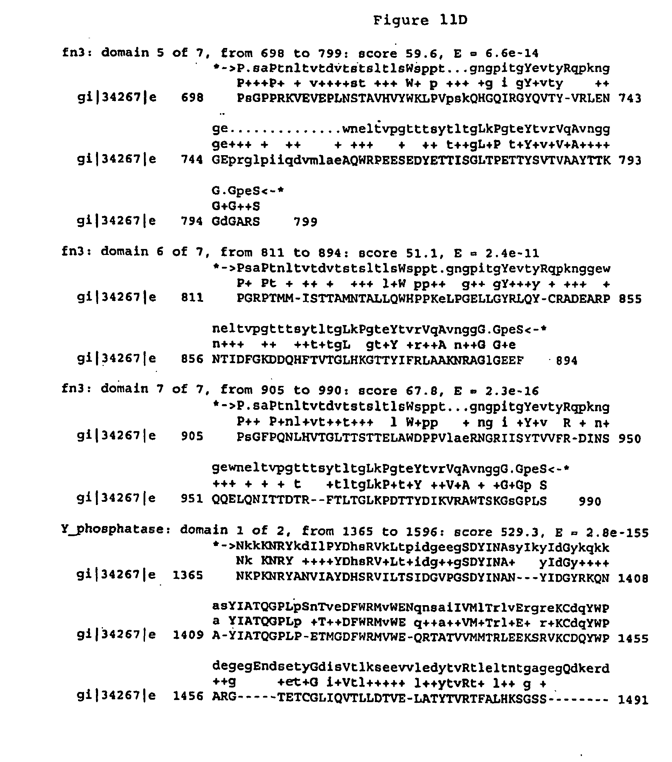

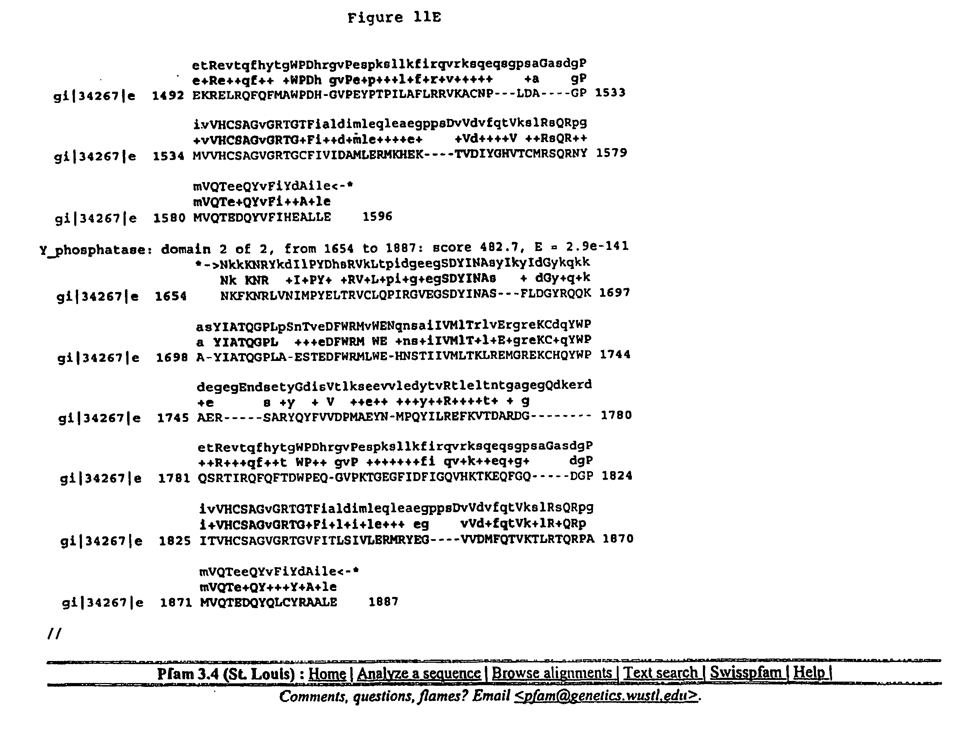

The term "PTP LAR" as used herein, refers to either the amino acid sequence provided in Figure 9, and fragments and derivatives thereof, provided that a functional activity is retained. Preferred functional activities of PTP LAR include, but are not limited to, the ability to inhibit/prevent tyrosine phosphorylation of β-catenin, the ability to prevent an increase (or lower) β-catenin free pools, and the ability to inhibit/prevent epithelial cell migration in vitro or in vivo. Specific fragments including functional domains envisioned to be included in the invention include those described in the Examples and figure 11, herein. In addition, other modifications, substitutions, and deletions are envisioned such that they do not abrogate a functional activity. These are discussed in more detail in the detailed description of the invention herein.

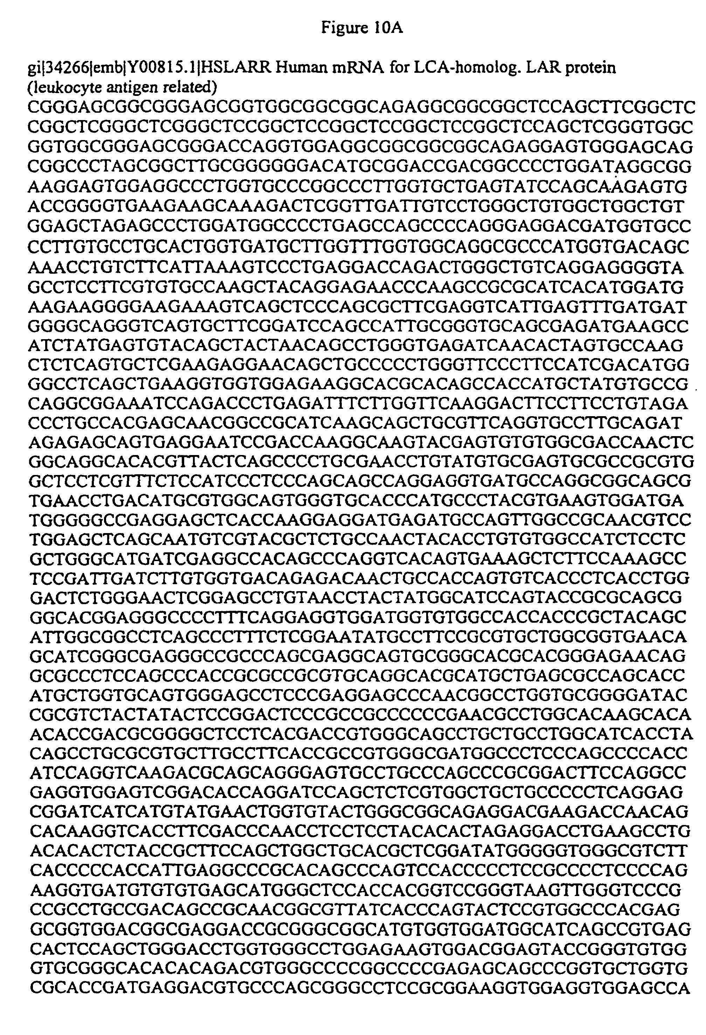

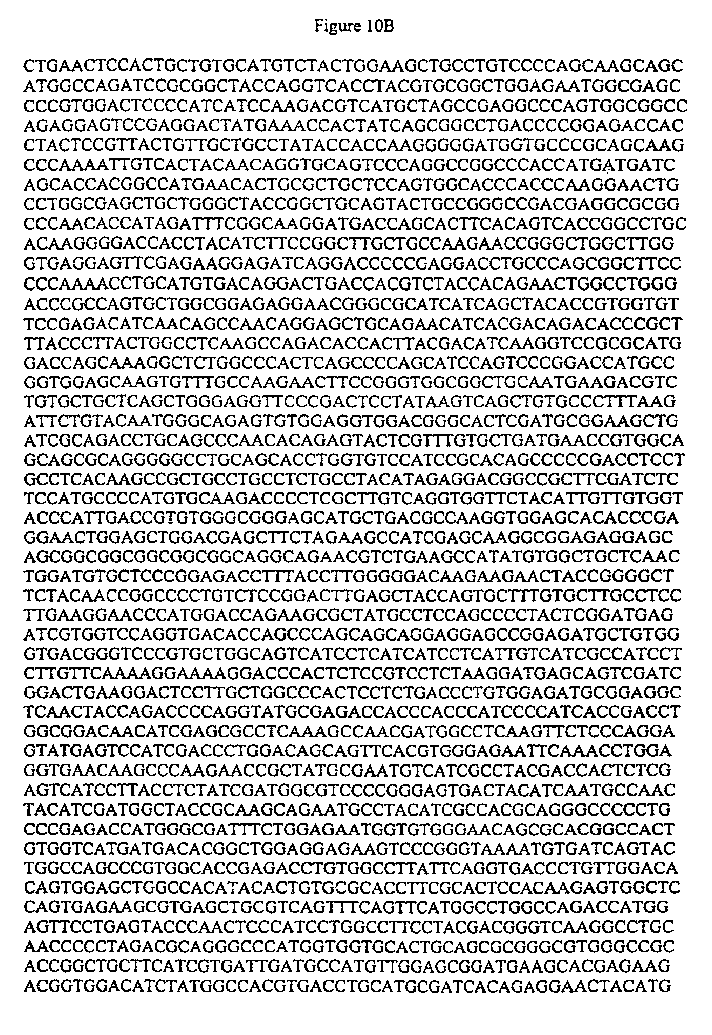

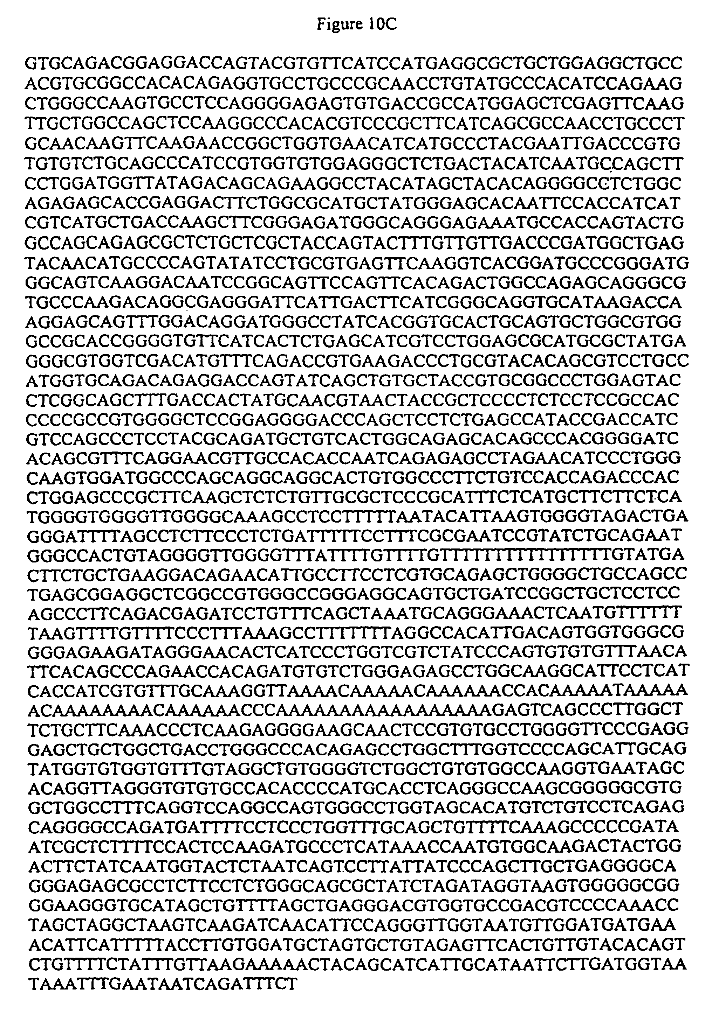

Further to the definition of "PTP LAR", there is also described herein a nucleic acid sequence encoding PTP LAR (figure 10). Similarly to above, fragments encoding functional domains of PTP LAR are also included, as are derivatives of the nucleic acid encoding PTP LAR or functional fragments thereof, as is described in more detail herein in the detailed description of the invention.

The term "pharmaceutically acceptable" or "pharmaceutical" as used herein refers to solutions or components of the pharmaceutical composition that do not prevent the therapeutic compound from exerting a therapeutic effect and do not cause unacceptable adverse side effects. Examples of pharmaceutically acceptable reagents are provided in *The United States Pharmacopeia The National Formulary,* United States Pharmacopeial Convention, Inc., Rockville, MD 1990 and *FDA Inactive Ingredient Guide* 1990, 1996 issued by the Division of Drug Information Resources (both are hereby incorporated by reference herein, including any drawings). Unacceptable side effects vary for different diseases. Generally, the more severe the disease the more toxic effects which will be tolerated. Unacceptable side effects for different diseases are known in the art.

The term "physiologically acceptable" defines a carrier or diluent that does not cause significant irritation to an organism and preferably does not abrogate the biological activity and properties of the compound.

The term "carrier" defines a chemical compound that facilitates the incorporation of a compound into cells or tissues. For example dimethyl sulfoxide (DMSO) is a commonly utilized carrier as it facilitates the uptake of many organic compounds into the cells or tissues of an organism.

The term "diluent" defines chemical compounds diluted in water (or another solvent) that will dissolve the compound of interest as well as stabilize the biologically active form of the compound. Many salts dissolved in buffered solutions are utilized as diluents in the art. One commonly used buffered solution is phosphate buffered saline because it mimics the salt conditions of human blood. Because buffer salts can control the pH of a solution at low concentrations, a diluent rarely modifies the biological activity of a compound.

The term "solvent" as used herein refers to a chemical compound that facilitates the solubilization of compounds of the invention. Examples of solvents include, but are not limited to, pharmaceutically acceptable alcohols, such as ethanol and benzyl alcohol; polyoxyhydrocarbyl compounds, sucn as poly(etnylene glycol); pharmaceutically acceptable surfactants such as CREMOPHOR® EL; polyglycolized lipids, such as GELUCIRE® and LABRASOL®; and pharmaceutically acceptable oils, such as miglyol 812.

The term "pharmaceutically acceptable alcohol" as used herein refers to alcohols that are liquids at about room temperature (approximately 20 •C). These include propylene glycol, ethanol, 2-(2-ethoxyethoxy)ethanol (TRANSCUTOL®, Gattefosse, Westwood, NJ 07675), benzyl alcohol, and glycerol.

The term "polyoxyhydrocarbyl compound" as used herein refers to a water soluble carbohydrate such as glucose, sucrose, maltotriose, and the like; water soluble carbohydrate derivatives such as gluconic acid and mannitol, and oligosaccharides; and water soluble polymers such as polyvinylpyrrolidone, poly(vinyl alcohol), and in particular, polyethers such as other polyoxyalkylenes including poly(ethylene glycol) or other water soluble mixed oxyalkylene polymers and the polymeric form of ethylene glycol. Although polyoxyhydrocarbyl compounds preferably contain more than one carbon, oxygen, and hydrogen atom, some molecules such as poly(ethylene imine) are also included.

A particularly preferred class of solubilizing polyoxyhydrocarbyl moieties comprises poly(ethylene glycol) (PEG) and PEG derivatives, such as PEG monomethyl ether. Other suitable PEG derivatives include PEG-silicon derived ethers. Many of these polymers are commercially available in a variety of molecular weights. Others may be conveniently prepared from commercially available materials, such as by coupling of amino-PEG moiety to a haloalkyl silyl or silane moiety.

Suitable PEGs may vary in molecular weight from about 200 g/mol to about 20,000 g/mol or more, more preferably 200 g/mol to 5,000 g/mol, even more preferably 250 g/mol to 1,000 g/mol, and most preferably 250 g/mol to 500 g/mol. The choice of a particular molecular weight may depend on the particular compound chosen and its molecular weight and degree of hydrophobicity, as well as the particular application for which the formulation is to be used.

The term "pharmaceutically acceptable surfactant" as used herein refers to a compound that can solubilize compounds of the invention into aqueous solutions, if necessary. Preferably for parenteral formulations, the surfactant is a non-ionic surfactant. Examples of pharmaceutically acceptable surfactants include POLYSORBATE 80 and other polyoxyethylene sorbitan fatty acid esters, glyceryl monooleate, polyvinyl alcohol, ethylene oxide copolymers such as PLURONIC® (a polyether) and TETRONIC® (BASF), polyol moieties, and sorbitan esters. Preferably ethoxylated castor oils, such as CREMOPHOR® EL, are used for the formulation of some compounds.

The term "ethoxylated castor oil" as used herein refers to castor oil that is modified with at least one oxygen containing moiety. In particular the term refers to castor oil comprising at least one ethoxyl moiety.

Further, the term "pharmaceutically acceptable surfactant" as used herein in reference to oral formulations, includes pharmaceutically acceptable non-ionic surfactants (for example polyoxyethylenepolypropylene glycol, such as POLOXAMER® 68 (BASF Corp.) or a mono fatty acid ester of polyoxyethylene (20) sorbitan monooleate (TWEEN® 80), polyoxyethylene (20) sorbitan monostearate (TWEEN® 60), polyoxyethylene (20) sorbitan monopalmitate (TWEEN® 40), polyoxyethylene (20) sorbitan monolaurate (TWEEN® 20) and the like); polyoxyethylene castor oil derivatives (for example, polyoxyethyleneglycerol-triricinoleate or polyoxyl 35 castor oil (CREMOPHOR® EL, BASF Corp.), polyoxyethyleneglycerol oxystearate (CREMOPHOR® RH 40 (polyethyleneglycol 40 hydrogenated castor oil) or CREMOPHOR® RH 60 (polyethyleneglycol 60 hydrogenated castor oil), BASF Corp.) and the like); or a pharmaceutically acceptable anionic surfactant.

The term "polyglycolized lipids" as used herein refers to mixtures of monoglycerides, diglycerides, or triglycerides and polyethyleneglycol monoesters and diesters formed by the partial alcoholysis of vegetable oil using PEG of 200 g/mol to 2,000 g/mol or by the esterification of fatty acids using PEG 200 g/mol to 2,000 g/mol and glycerols. Preferably these include GELUCIRE® 35/10, GELUCIRE® 44/14, GELUCIRE® 46/07, GELUCIRE® 50/13, GELUCIRE® 53/10, and LABRASOL®.

The term "pharmaceutically acceptable oils" as used herein refers to oils such as mineral oil or vegetable oil (including safflower oil, peanut oil, and olive oil), fractionated coconut oil, propylene glycol monolaurate, mixed triglycerides with caprylic acid and capric acid, and the like. Preferred embodiments of the invention feature mineral oil, vegetable oil, fractionated coconut oil, mixed triglycerides with caprylic acid, and capric acid. A highly preferred embodiment of the invention features Miglyol® 812 (available from Huls America, USA).

In preferred embodiments of methods of treating or preventing a disease or disorder PTP LAR is provided as a recombinant protein, a recombinant gene, or as part of a recombinant cell. Methods to do this are well-known to one of ordinary skill in the art, and examples are discussed further herein in the detailed description of the invention.

The term "recombinant" as used herein refers to the manipulation of genes, proteins, and cells in the laboratory that is standard in the art. The resulting gene protein, or call may be identical to the gene, protein, or cell in its native host, or it may have been modified in some way for ease of manipulation, or to place it in an appropriate vector, or to allow it to more easily detected, or to be less easily degraded, for example. Some aspects of the invention include recombinant fragments of PTP LAR manipulated for gene therapy applications in vitro and in vivo.

In other preferred embodiments of methods of treating or preventing a disease or disorder, PTP LAR modulates epithelial cell migration *in vitro* or *in vivo,* and preferably inhibits epithelial cell migration *in vivo* or *in vitro.* PTP LAR may also (or instead) modulate tyrosine phosphorylation of β-catenin *in vitro* or *in vivo,* and preferably inhibits tyrosine phosphorylation of β-catenin *in vitro* or *in vivo*. Finally, PTP LAR may also (or instead) modulate the free pool of β-catenin *in vitro* or *in vivo,* and preferably decreases the level of the free pool of β-catenin *in vitro* or *in vivo.*

The term "modulates" refers to the ability of PTP LAR or a compound to alter epithelial cell migration, β-catenin tyrosine phosphorylation, or levels of β-catenin free pools. A modulator preferably inhibits, although in some circumstances it may be preferable to increase these functions.

The term "modulates" also refers to increasing or decreasing the probability that a complex forms between the PTP LAR and a natural binding partner. A modulator preferably increases the probability that such a complex forms between PTP LAR and a natural binding partner, or decreases the probability that a complex forms between PTP LAR and a natural binding partner. The term "complex" refers to an assembly of at least two molecules bound to one another.

The term "natural binding partner" refers to polypeptides, lipids, small molecules, or nucleic acids that bind to PTP LAR. A change in the interaction between PTP LAR and a natural binding partner can manifest itself as an increased or decreased probability that the interaction forms, or an increased or decreased concentration of PTP LAR/natural binding partner complex, as well as changes in tyrosine phosphorylation of β-catenin, or levels of the free pools of β-catenin.

In a second aspect, the invention features a method for prognosis of a disease or disorder by detection of PTP LAR in a sample, wherein the method comprises: (a) contacting the sample with a nucleic acid probe which hybridizes under hybridization assay conditions to a nucleic acid target region of PTP LAR, the probe comprising a nucleic acid sequence encoding PTP LAR, fragments thereof, or the complements of said sequences and fragments; and (b) detecting the presence or amount of the probe:target region hybrid as an indication of the prognosis of the disease or disorder. In preferred embodiments, the disease or disorder is selected from the group consisting of aberrant wound healing, cancer, and metastases.

An alternate method for diagnosis of a disease or disorder by detection of PTP LAR in a sample comprises: (a) comparing a nucleic acid target region encoding PTP LAR in a sample with a control nucleic acid target region encoding PTP LAR; and (b) detecting differences in sequence or amount between said target region and said control target region, as an indication of said prognosis of said disease or disorder. Preferably the disease or disorder is selected from the group consisting of aberrant wound healing, cancer, and metastases.

An additional method for diagnosis of a disease or disorder by detection of PTP LAR in a sample comprises: (a) contacting the sample with an antibody which hybridizes to PTP LAR; and (b) detecting the presence or amount of the antibody:PTP LAR complex as an indication of the prognosis of the disease or disorder. Preferably, the antibody is either a polyclonal or a monoclonal antibody, or is from a hybridoma. Preferably the disease or disorder is selected from the group consisting of aberrant wound healing, cancer, and metastases.

The term "prognostic" as used herein means indicating the liklihood of a cure, or the liklihood of being amenable to treatment, or the liklihood of metastases, for example. Thus, it relates to diseases progression, and disease cure, and disease treatment outcome.

The term "contacting" as used herein refers to mixing a solution comprising the test compound with a liquid medium bathing the cells or the proteins of the methods. The solution comprising the compound may also comprise another component, such as dimethyl sulfoxide (DMSO), which facilitates the uptake of the test compound or compounds into the cells of the methods. The solution comprising the test compound may be added to the medium bathing the cells by utilizing a delivery apparatus, such as a pipet-based device or syringe-based device.

By hybridization assay conditions is meant hybridization assay conditions at least as stringent as the following: hybridization in 50% formamide, 5X SSC, 50 mM NaH₂PO₄, pH 6.8, 0.5% SDS, 0.1 mg/mL sonicated salmon sperm DNA, and 5X Denhart solution at 42 °C overnight; washing with 2X SSC, 0.1% SDS at 45 °C; and washing with 0.2X SSC, 0.1% SDS at 45 °C. Under some of the most stringent hybridization assay conditions, the second wash can be done with 0.1X SSC at a temperature up to 70 °C (Berger et al. (1987) Guide to Molecular Cloning Techniques pg 421, hereby incorporated by reference herein including any figures, tables, or drawings.). However, other applications may require the use of conditions falling between these sets of conditions. Methods of determining the conditions required to achieve desired hybridizations are well-known to those with ordinary skill in the art, and are based on several factors, including but not limited to, the sequences to be hybridized and the samples to be tested.

By "nucleic acid target region" is meant a fragment of the PTP LAR gene that is preferably unique, so that a probe hybridizes to only the PTP LAR gene. By unique is meant that the sequence is not present in any other known protein. Techniques to do this are well known to those with ordinary skill in the art. The "control" nucleic acid target region refers to the identical region of nucleic acid in the known PTP LAR gene (figure 10) as compared to that in the PTP LAR gene in the sample that is being tested. Under some disease conditions, it is anticipated that the sequence will be different in the PTP LAR gene from the sample than in the control sequence. Alternatively, the amount of PTP LAR gene in the sample may be higher or lower than that found in normal sample of cells or tissues.

The term "detecting" as used herein includes any method to detect a protein, antibody, or nucleic acid. These methods are well-known in the art and many are discussed herein. In particular, these include such methods as ELISA, PCR, radio-labels, etc.

In a third aspect, the invention features a method for identifying one or more compounds that modulate epithelial cell migration, comprising: (a) contacting tyrosine phosphorylated β-catenin with one or more potential compounds; (b) monitoring a change in the phosphorylation level of said tyrosine phosphorylated β-catenin to identify said one or more compounds that modulate said epithelial cell migration. In preferred embodiments, the one or more compounds are selected from the group consisting of indolinones, quinazolines, quinoxalines, and tyrphostins.

An alternative method for identifying one or more compounds that modulate PTP LAR activity, comprises: (a) contacting PTP LAR with one or more potential compounds; (b) measuring the activity of PTP LAR; and (c) determining whether the one or more potential compounds modulates the activity of said PTP LAR. The activity measured is phosphotyrosine phosphatase LAR activity. In preferred embodiments, the method comprises the addition of a natural binding partner binding to part (a). Preferably, the natural binding partner is selected from the group consisting of β-catenin and plakoglobin and the one or more compounds are selected from the group consisting of indolinones, quinazolines, quinoxalines, and tyrphostins.

Another alternate method for identifying one or more compounds that modulate PTP LAR activity in cells, comprises: (a) expressing PTP LAR in said cells; (b) contacting said cells with one or more potential compounds; and (c) monitoring a change in cell migration or the interaction between said PTP LAR and a natural binding partner to identify said one or more compounds that modulate said PTP LAR activity. Preferably, the natural binding partner is selected from the group consisting of β-catenin, and plakoglobin, and the one or more compounds are selected from the group consisting of indolinones, quinazolines, quinoxalines, and tyrphostins.

The term "compound" preferably refers to a non-peptide organic molecule, and most preferably refers to a non-peptide synthetic organic molecule. The term "non-peptide molecule" refers to a compound that is not a polymer of amino acids. A non-peptide molecule preferably does not contain chemical moieties that hydrolyze in physiological conditions, e.g. a peptidomimetic. Examples of compounds are included in the Description of the Invention, herein. Preferably, such molecules have a molecular weight less than 3,000.

The term "expressing" as used herein preferably refers to the production of PTP LAR from a nucleic acid vector within a cell. The nucleic acid vector is transfected into cells using well known techniques in the art as described herein. "At higher levels" can indicate that PTP LAR is normally not expressed at all, or that expression occurs normally but that the level is higher. Levels of expression (and comparisons) can be determined by methods well-known in the art (and examples are described herein). Higher expression is preferably 2-fold, more preferably 5-fold or more. Lower expression is the opposite.

The term "nucleic acid vector" relates to a single or double stranded circular nucleic acid molecule that can be transfected into cells and replicated within or independently of a cell genome. A circular double stranded nucleic acid molecule can be cut and thereby linearized upon treatment with restriction enzymes. An assortment of nucleic acid vectors, restriction enzymes, and the knowledge of the nucleotide sequences cut by restriction enzymes are readily available to those skilled in the art. A nucleic acid molecule encoding a chimeric receptor can be inserted into a vector by cutting the vector with restriction enzymes and ligating the two pieces together.

The term "transfecting" defines a number of methods to insert a nucleic acid vector or other nucleic acid molecules into a cell. These methods involve a variety of techniques, such as treating the cells with high concentrations of salt, an electric field, detergent, or DMSO to render the outer membrane or wall of the cells permeable to nucleic acid molecules of interest or use of various viral transduction strategies.

The term "monitoring" refers to observing the effect of contacting the cells with substances including, but not limited to, one or more compounds and PTP LAR. The effect can be manifested in cell phenotype or migration, or in the interaction between PTP LAR and a natural binding partner. More preferably, the effect to be monitored is the phosphorylation level of β-catenin or the level of free β-catenin pools.

The term "effect" preferably describes a change or an absence of a change in cell phenotype. "Effect" can also describe a change or an absence of a change in the catalytic activity of PTP LAR. "Effect" can also describe a change or an absence of a change in an interaction between PTP LAR and a natural binding partner, such as β-catenin or plakoglobin. More preferably, "effect" describes a change or an absence of a change in the phosphorylation level of β-catenin, the level of free β-catenin pools, or the epithelial cell migration.

The term "cell phenotype" refers to the outward appearance of a cell or tissue or the function of the cell or tissue. Examples of cell phenotype include, but are not limited to, cell size (reduction or enlargement), cell shape, cell proliferation (increased numbers of cells), cell differentiation (changes in physiological state), cytotoxicity (cell death), cell survival, apoptosis (programmed cell death), or the utilization of a metabolic nutrient (*e.g.*, glucose uptake). Most preferably, cell phenotype refers to the presence or absence of epithelial cell migration-the taking on the fibroblastoid shape. Changes or the absence of changes in cell phenotype are readily measured by techniques known in the art.

The term "catalytic activity", in the context of the invention, defines the rate at which PTP LAR dephosphorylates a substrate. Catalytic activity can be measured, for example, by determining the amount of a substrate converted to a dephosphorylated product as a function of time. Catalytic activity can be measured by methods of the invention by holding time constant and determining the concentration of a dephosphorylated substrate after a fixed period of time.
Dephosphorylation of a substrate occurs at the active-site of PTP LAR. The active-site is normally a cavity in which the substrate binds to PTP LAR and is dephosphorylated. Methods for determining the dephosphorylation of β-catenin are described herein. Those in the art would be able to envision other appropriate methods.

In preferred embodiments, the one or more compounds modulate, and preferably inhibit, the epithelial cell migration *in vitro.* Alternatively, the one or more compounds modulate, and preferably inhibit, tyrosine phosphorylation (or dephosphorylate) of β-catenin *in vitro.* Alternatively, the one or more compounds modulate, and preferably decrease the levels of β-catenin free pools, *in vitro.* Finally, in other preferred embodiments, the one or more compounds is selected from the group consisting of quinazolines, indolinones, quinoxalines, and tyrphostins.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

The summary of the invention described above is not limiting and other features and advantages of the invention will be apparent from the following detailed description of the invention, and from the claims.

### BRIEF DESCRIPTION OF THE DRAWINGS

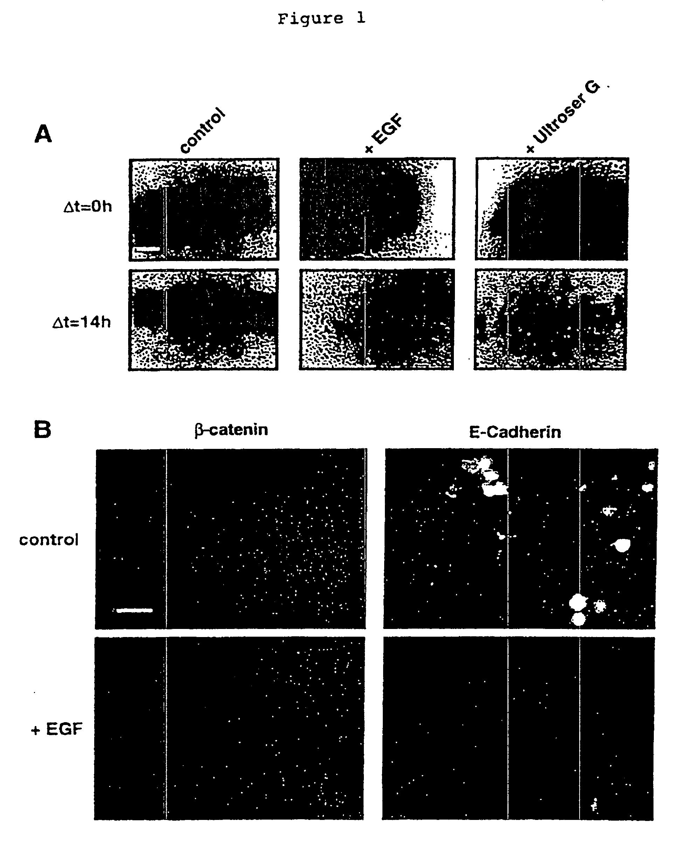

Figures 1A and 1B show the migration of epithelial NBT II cells under varying conditions. Figure 1A shows an *in vitro* wound assay. Twenty-four hours after plating, the confluent cell monolayer was scratched as described in the Examples. EGF (100 ng/mL) or Ultroser G (2%) were added. Wound closure was documented by photography. The calibration bar indicates 80 µm.
Figure 1B shows the results of immunofluorescence studies. Twenty-four hours after plating, EGF (100 ng/mL) was added to the subconfluent cells. Six hours later the cells were fixed for subsequent immunolabeling, and images were recorded by conventional fluorescence microscopy. The upper panel shows untreated controls, while the lower panel shows cells treated with EGF. The green fluorescence indicates the presence of E-cadherin, while the red fluorescence represents β-catenin. Controls incubated without primary antibody remained negative for a fluorescence signal. The calibration bar indicates 20 µm.

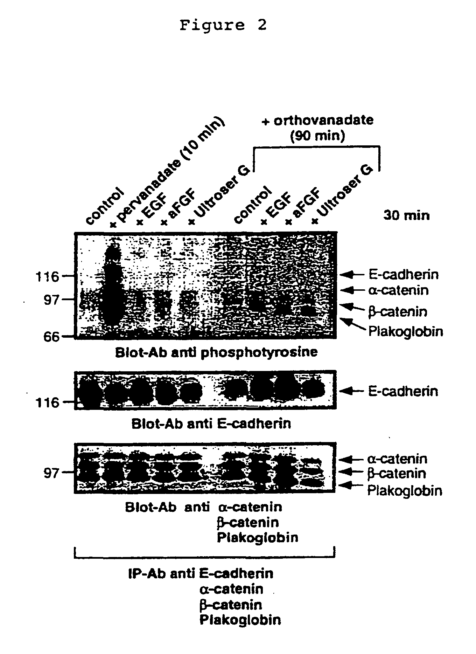

Figure 2 shows the tyrosine phosphorylation of β-catenin and plakoglobin during epithelial cell migration. NBT II cells were plated at a density of 1x10⁴ cells/cm². Twenty-four hours later growth factors were added to induce migration (EGF: 100 ng/mL; aFGF: 30 ng/mL including 50 µg/mL heparin; Ultroser G: 2%). Ninety minutes before lysis, Na-orthovanadate (1 mM) was added (right) or not (left). As a positive control, cells were treated for 10 minutes with pervanadate. After lysis the cadherin/catenin complex was immunoprecipitated and precipitates were separated by SDS-PAGE. Tyrosine phosphorylation levels were analyzed by Western blotting with an anti-phosphotyrosine antibody (top). The membranes were reprobed with specific antibodies to E-cadherin (middle) or the catenins (bottom) to assure equal amounts of precipitated proteins. Highly tyrosine phosphorylated β-catenin (as hereafter pervanadate treatment) was not efficiently immunodecorated in the reblot. Arrows indicate the proteins of interest. Molecular mass standards in kilodaltons are shown on the left.

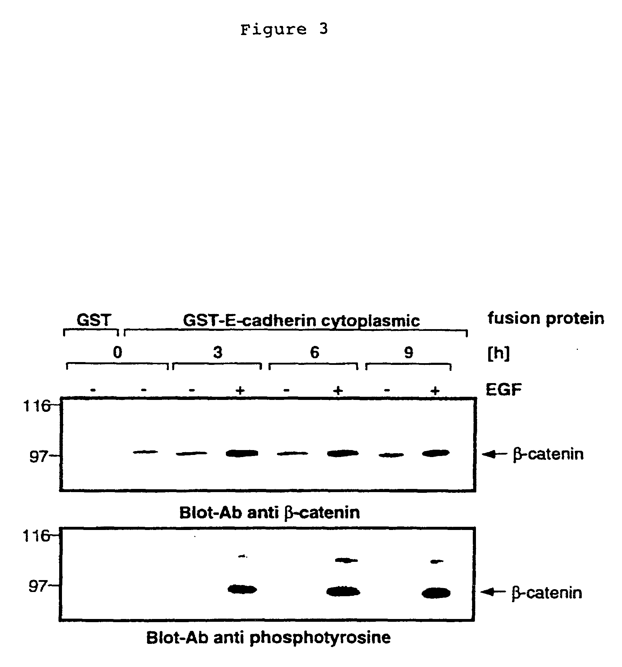

Figure 3 shows that during epithelial cell migration, the free, uncomplexed pool of β-catenin is increased and correlates with enhanced tyrosine phosphorylation. NBT II cells were plated at a density of 1x10⁴ cells/cm². Twenty-four hours later EGF (100 ng/mL) was added for the indicated time intervals. 30 minutes before lysis Na-orthovanadate (1 mM) was added. Subsequently affinity precipitations were performed with 5 µg of a GST/E-cadherin cytoplasmic protein or GST in a three-fold molar excess. Proteins were separated by SDS-PAGE, and the levels of free, uncomplexed β-catenin were analyzed by Western blotting with an anti-β-catenin-antibody (top). Western blotting with an anti-phosphotyrosine antibody shows the increase in the level of tyrosine phosphorylation in free, uncomplexed β-catenin (bottom). Note that in comparison to Figure 2, cells were pretreated for only 30 minutes with Na-orthovanadate. Arrows indicate the proteins of interest. Molecular mass standards in kilodaltons are shown on the left.

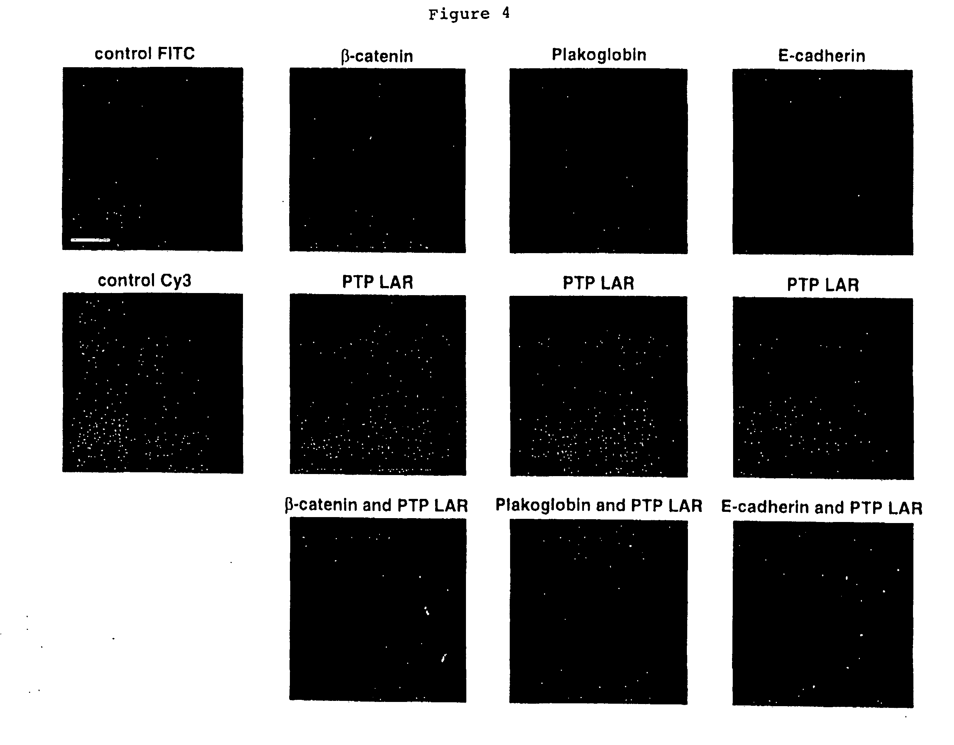

Figure 4 shows that members of the cadherin/catenin complex colocalize with PTP LAR in NBT II cells. Twenty-four hours after plating, confluent monolayers of NBT II cells were fixed, and were processed for indirect Immunofluorescence. Cells were labeled with antibodies against either β-catenin, plakoglobin or E-cadherin (green fluorescence, upper panel), and simultaneously with an antibody against PTP LAR (red fluorescence, middle panel), as indicated. Laser confocal fluorescence images show the colocalization (yellow superimposition, lower panel) of PTP LAR with the cadherin/catenin complex at adherens junctions. Controls without primary antibodies remained negative for a fluorescence signal. The calibration bar indicates 20 µm.

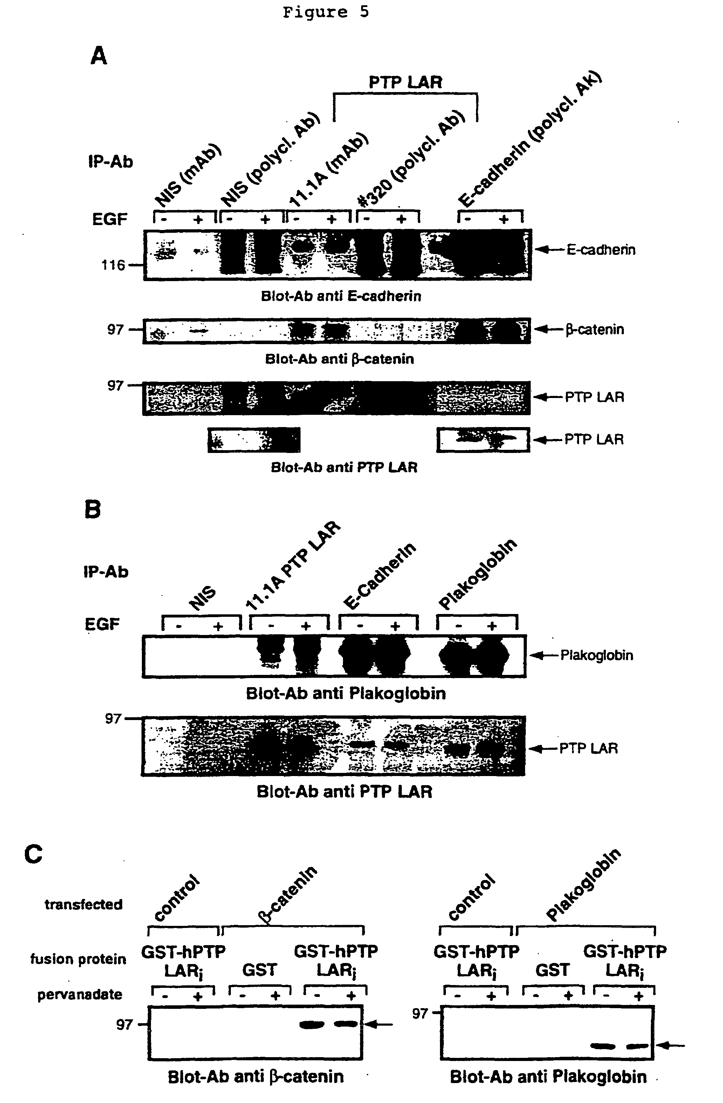

Figures 5A, 5B, and 5C show that members of the cadherin/catenin-complex associate with PTP LAR. Figures 5A and 5B show the association of the cadherin/catenin-complex with PTP LAR in intact cells. Human epithelial MCF7 cells were plated at 1x10⁴ cells/cm² and stimulated 24 h later with EGF (100 ng/mL) as indicated. Cells were lysed and were immunoprecipitated with antibodies against PTP LAR (monoclonal antibody 11.1A, rabbit polyclonal antiserum #320) or members of the cadherin/catenin-complex as indicated. NIS indicates the non-immune-serum control. Proteins were separated by SDS-PAGE and analyzed by Western blotting with antibodies against E-cadherin (figure 5A, top), β-catenin (figure 5A, middle), plakoglobin (figure5B, top) or PTP LAR (Figures 5A and 5B, bottom; Figure 5A, long exposures were shown on the very bottom). Arrows indicate the proteins of interest. Molecular mass standards in kilodaltons are shown on the left.
Figure 5C shows the *in-vitro* association of β-catenin and plakoglobin with PTP LAR. β-catenin and plakoglobin were transiently overexpressed in human embryonic kidney 293 fibroblasts. After 24 h of serum starvation, the cells were stimulated with pervanadate for 10 minutes before lysis. Equal amounts of lysats were incubated with the GST-PTP LAR cytoplasmic fusion protein (GST-PTP LARᵢ) or a threefold molar excess of GST. Lysates of control vector transfected 293 cells were incubated with the GST-PTP LAR cytoplasmic fusion protein (GST-PTP LARᵢ). Complexes were immobilized on glutathione-sepharose, and precipitates were separated by SDS-PAGE. Bound proteins were analyzed by Western blotting with an anti-β-catenin antibody (left) or an antibody against plakoglobin (right). Arrows indicate the proteins of interest. Molecular mass standards in kilodaltons are shown on the left.

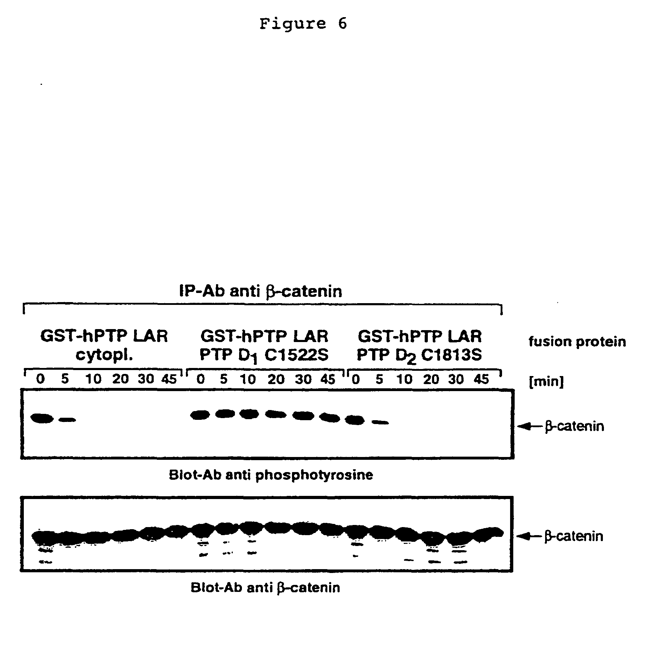

Figure 6 shows that β-catenin is a substrate of PTP LAR *in vitro.* β-catenin was transiently overexpressed in human embryonic kidney 293 fibroblasts. After 24 h of serum starvation, the cells were stimulated with pervanadate before lysis. β-catenin was subsequently immunoprecipitated with an anti-β-catenin antibody, and precipitates were incubated for the indicated time intervals with GST-PTP LAR cytoplasmic fusion protein (left), GST-PTP LAR PTP D₁ C1522S-mutant (middle) or GST-PTP LAR PTP D2 C1813S-mutant (right). Reactions were terminated after the indicated periods of time, and proteins were separated by SDS-PAGE. Tyrosine phosphorylation levels of β-catenin were analyzed by Western blotting with an anti-phosphotyrosine antibody (top). Western blotting with an anti-β-catenin antibody revealed that equal amounts of β-catenin were immunoprecipitated (bottom). Arrows indicate the proteins of interest.

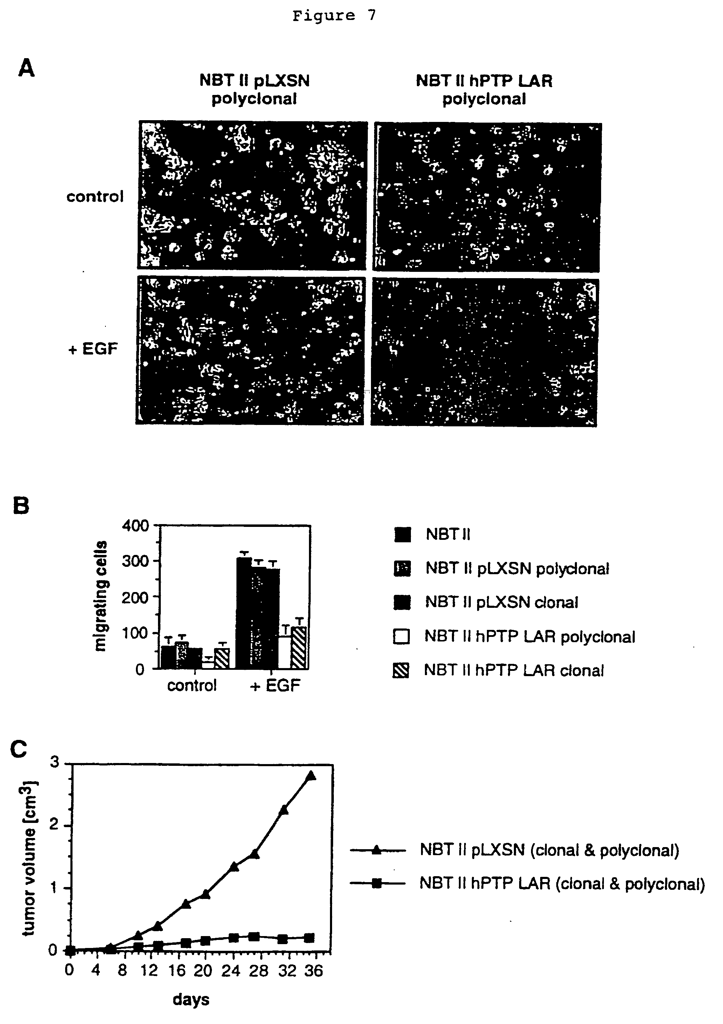

Figures 7A, 7B, and 7C show that the ectopic overexpression of human PTP LAR inhibits epithelial cell migration and tumor formation of rat NBT II cells in nude mice. Figures 7A and 7B show a scatter assay of NBT II pLXSN and NBT II hPTP LAR cells. In Figure 7A, NBT II cells infected with the empty vector pLXSN (left) or human PTP LAR (right) were plated at 1x10⁴ cells/cm² and stimulated 24 h later with EGF (100 ng/mL) as indicated. After 7 h, the migration morphology was documented by photography.
Figure 7B shows a quantification of the scatter assay (+/- standard deviation). To this end, 1000 cells per dish from randomly chosen microscopic fields were counted; assays were performed in triplicate. A cell was judged as a migrating cell when it had changed from a cobblestone-like, epithelial morphology to a migrating, fibroblastoid phenotype.
Figure 7C shows tumor formation in nude mice by NBT II pLXSN and NBT II nPTP LAR cells. NBT II cells, infected with the empty vector pLXSN (filled triangle) or human PTP LAR (filled square), were injected (2x10⁶/50 µL) subcutaneously into the flank region of Swiss nude mice. Tumor formation was monitored. The graph shows the average tumor volume of 3 tumors per polyclonal and clonal cell line, respectively.

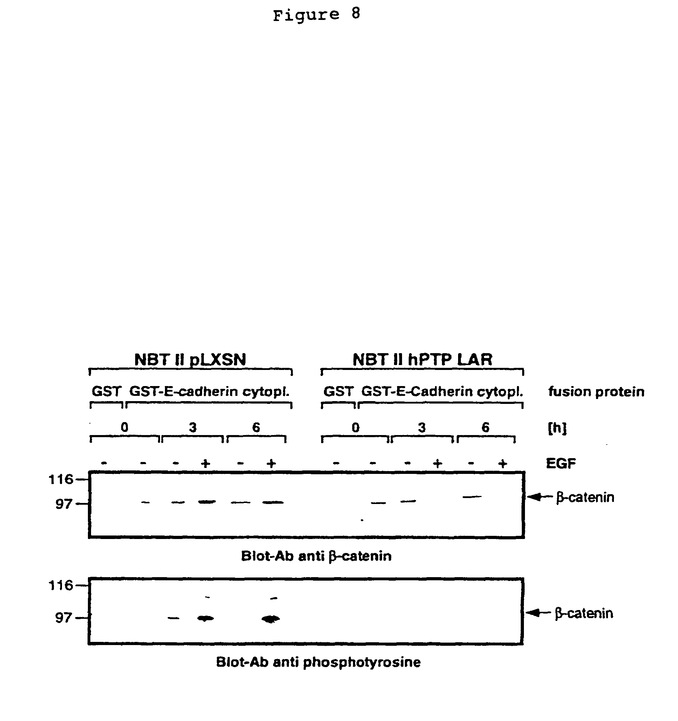

Figure 8 shows that the ectopic expression of human PTP LAR in NBT II cells inhibits the phosphorylation of tyrosine residues of β-catenin, as well as the increase of the free pool of β-catenin. NBT II cells infected with the empty vector pLXSN (left) or human PTP LAR (right) were plated at 1x20⁴ cells/cm² and stimulated after 24 h with EGF (100 ng/mL) for the indicated time intervals. Na-orthovanadate pretreatment was omitted to avoid irreversible inhibition of protein tyrosine phosphatase activity. Cells were lysed and *in vitro* associations were performed with 5 µg of the GST-E-cadherin cytoplasmic protein or GST in a three-fold molar excess. Proteins were separated by SDS-PAGE, and the levels of free, uncomplexed β-catenin were analyzed by Western blotting with an anti-β-catenin antibody (top). Western blotting with an anti-phosphotyrosine antibody shows the increased tyrosine phosphorylation in free, uncomplexed β-catenin only in the control-infected NBT II cell line (bottom). Arrows indicate the proteins of interest. Molecular mass standards in kilodaltons are shown on the left.
Figure 9 shows the amino acid sequence of the PTP LAR preprotein extracted from the EMBL database.
Figures 10A, 10B, and 10C show the nucleic acid sequence of PTP LAR extracted from the EMBL database.
Figures 11A, 11B, 11C, 11D, and 11E show the output resulting from a search in Pfam HMM with the PTP LAR amino acid sequence. The site is http://pfam.wustl.edu/cgi-bin/nph-hmmsearch. The phosphatase domains were "green" indicating a significant E-value.

### DETAILED DESCRIPTION OF THE PREFERRED EMBODIMENTS

Cadherin/catenin-complex mediated cell-cell adhesion as well as adhesion-independent functions of catenins have been implicated in the modulation of multicellular differentiation, proliferation, and malignant transformation of epithelial cells (Marrs, *et al*. (1996) *Int. Rev. Cytol.* **165,** 159-205; Birchmeier, *et al*. (1995) *Ciba F. Symp*. **189**, 124-141; Huber, *et al*. (1996) *Curr. Opin. Cell Biol*. **8**, 685-691). Cell migration is an essential process during embryonic development and in epithelial regeneration of adult organisms, for example in wound healing, and requires precise control, which is altered or lost when tumor cells become invasive and metastatic. As part of the invention, distinct mechanisms regulating epithelial cell migration have been defined at the cellular and biochemical level.
Furthermore, the impact of tyrosine phosphorylation of β-catenin, and its regulation by tyrosine kinases and the protein tyrosine phosphatase LAR, has been characterized in motile cells.

Growth factors such as EGF, aFGF or hepatocyte grown factor/scatter factor have been shown to induce migration of epithelial cells (Manske, *et al*. (1994) *Int. Rev. Cytol.* **155**, 49-96). A correlation has been suggested between migration and tyrosine phosphorylation of β-catenin (Behrens, *et al*. (1993) *J. Cell Biol*. **120**, 757-766; Shibamoto, *et al*. (1994) *Cell Adhes. Commun*. **1**, 295-3), but its significance remained unclear. Inhibition of tyrosine kinase signaling inhibits epithelial cell migration of NBT II cells. RTKs like the EGF receptor (Hoschuetzky, *et al*. (1994) *J. Cell Biol.* **127**, 1375-1380) or cytoplasmic tyrosine kinases like Src are able to mediate tyrosine phosphorylation of β-catenin (Hamaguchi, *et al*. (1993) *EMBO J.* **12**, 307-314). In NBT II cells a dominant-negative c-Src mutant was able to inhibit migration (Rodier, *et al*. (1995) *J. Cell. Biol*. **131**, 761-773).

The invention is drawn, *inter alia,* to the finding that after induction of migration, the pool of free, uncomplexed β-catenin is increased, and that this increase correlates with enhanced β-catenin tyrosine phosphorylation. Therefore, tyrosine phosphorylation may result in a reduced interaction of β-catenin with both E-cadherin and the actin-cytoskeleton. Since the integrity of the cadherin/catenin-complex is essential for strong cell-cell adhesion, this results in an overall decrease in intercellular contacts. Interestingly, a fusion protein of E-cadherin and α-catenin was reported to mediate the interaction of E-cadherin with the cytoskeleton independent of β-catenin. However, these cells were no longer capable of migration (Nagafuchi, *et al*. (1994) *J. Cell Biol*. **127**, 235-245). Thus, tyrosine phosphorylation of β-catenin may lead to disruption of the contact between E-cadherin and the cytoskeleton and to an increased pool of free β-catenin. This suggests a function for β-catenin independent of cadherin-mediated cell adhesion.

Besides their role at adherens junctions, a signaling function of β-catenin, or its *Drosophila* homologue Armadillo, was shown to be essential for normal embryonic development in *Drosophila* and *Xenopus.* Interference with the signaling function of free, uncomplexed β-catenin abolished proper vertebrate development (Miller, *et al*. (1996) *Genes Dev.* **10,** 2527-2539). Subsequent studies in *Drosophila* and *Xenopus* led to the discovery of a signaling cascade that regulates the cytoplasmic pool of β-catenin.

Without a signal, β-catenin is localized mainly at adherens junctions, and any free β-catenin is downregulated in a ubiquitin-dependent manner (Aberle, *et al*. (1997) *EMBO J.* **16,** 3797-3804). An extracellular signal such as Wnt or Wingless (Peifer, *et al*. (1994) *Development* **120,** 369-380; van Leeuwen, *et al*. (1994) *Nature* **368**, 342-344) leads via the receptor Frizzled (Bhanot, *et al*. (1996) *Nature* **382**, 225-230) to an inhibition of GSK3/Zeste White 3 activity, thus stabilizing β-catenin/Armadillo in its free form (Yost, *et al*. (1996) *Genes Dev.* **10**, 1443-1454; Papkoff, *et al*. (1996) *Mol. Cell. Biol*. **16**, 2128-2134) by inhibiting APC- and ubiquitin-dependent degradation (Munemitsu, *et al*. (1995) *Proc. Natl. Acad. Sci USA* **92**, 3046-3050; Aberle, *et al*. (1997) *EMBO J.* **16**, 3797-3804).

In the instant invention, EGF treatment resulted in an increased pool of free β-catenin in the cytoplasm and the nucleus in migrating cells, suggesting another way of regulating the free pool of β-catenin during epithelial cell migration beside Wnt/Wingless signaling. Tyrosine phosphorylation of β-catenin may also stabilize free β-catenin, since the tyrosine phosphorylated form of the homologous plakoglobin no longer associated with APC (Shibata, *et al*. (1994) *Biochem. Biophys. Res. Comm*. **203**, 519-522), thereby possibly preventing APC-mediated degradation. In addition, the free pool of β-catenin had an increased phosphotyrosine content during EGF-dependent epithelial cell migration. Additionally, EGF also inhibits GSK3 activity (Eldar-Finkelman, *et al*. (1995) *J. Biol. Chem*. **270**, 987-990). Tyrosine phosphorylation of β-catenin has been shown to correlate with carcinoma formation and tumor invasion (Sommers, *et al*. (1994) *Cancer Res.* **54**, 3544-3552). Only free β-catenin or plakoglobin is able to associate with members of the high mobility group (HMG; Grosschedl, *et al*. (1994) *Trends Genet.* **10,** 94-100) family of transcription factors, namely LEF1 and TCF3, TCF4 (Behrens, *et al*. (1996) *Nature* **382**, 638-642; Huber, *et al*. (1996) *Mech. Develop.,* **59,** 3-10; Molenaar, *et al*. (1996) *Cell* **86,** 391-399; Korinek, *et al*. (1997) *Science* **275**, 1784-1787; Morin, *et al*. (1997) *Science* **275**, 1787-1790). However, β-catenin and plakoglobin differ in their nuclear translocation and complexing with LEF1; LEF1-dependent transactivation is preferentially driven by β-catenin (Simcha, *et al*. (1998) *J. Cell Biol*. **141**, 1433-1448). The transcription factors LEF1 and TCF are able to induce dorsal mesoderm when expressed together with β-catenin in *Xenopus* embryos (Huber, *et al*. (1996) *Mech. Develop.,* **59,** 3-10; Behrens, *et al*. (1996) *Nature* **382,** 638-642; Molenaar, *et al*. (1996) *Cell* **86,** 391-399). Data from LEF1-deficient and LEF1-transgenic mice demonstrate an important role of this transcription factor during EMT since it is normally upregulated during EMT and inductive processes between mesenchyme and epithelium (van Genderen, *et al*. (1994) *Genes Dev.* **8**, 2691-2703; Zhou, *et al*. (1995) *Genes Dev.* **9**, 570-583; Kratochwil, *et al*. (1996) *Genes Dev.* **10**, 1382-1394). This suggests that a common regulatory system exists that coordinates the expression of genes for mesenchymal and epithelial phenotypes during embryonic development as well as in epithelial cell migration. During EMT or cell migration this common regulator would switch off expression of epithelial genes while switching on genes for the mesenchymal phenotype. The β-catenin/LEF1-complex may represent this common regulator and molecules, which control the free pool of β-catenin may be essential for proper development or wound healing.

PTPs were proposed to play an important role in the regulation of cell-cell contacts since treatment with Na-orthovanadate, a potent inhibitor of phosphatase activity, diminished normal cell contact inhibition in epithelial cells and led to increased tyrosine phosphorylation at adherens junctions (Volberg, *et al*. (1992) *EMBO J.* **11**, 1733-1742). PTPs may therefore act as steady state equilibrium antagonists of TKs for regulatory events at adherens junctions. Consistent with these findings we show that tyrosine phosphorylation of β-catenin and plakoglobin in migrating epithelial cells was significantly increased after pretreatment with Na-orthovanadate.

PTP LAR was recently shown to interact and colocalize at focal adhesions with LIP-1 (LAR interacting protein-1) and the multidomain protein Trio. However, neither of these proteins appears to be a substrate for PTP LAR (Serra-Pagès, *et al*. (1995) *EMBO J.* **14,** 2827-2838; Debant, *et al*. (1996) *Proc. Natl. Acad. Sci. USA* **93**, 5466-547). Moreover, a PTP LAR-like PTP was reported to interact with the cadherin/catenin-complex at adherens junctions of neurosecretory PC12 cells (Kypta, *et al*. (1996) *J. Cell Biol*. **134**, 1519-1529).

We demonstrate here the colocalization and interaction of PTP LAR with the cadherin/catenin-complex in epithelial cells. Furthermore, we show that only β-catenin and plakoglobin are able to associate directly with PTP LAR and we present evidence that β-catenin is a substrate of PTP LAR. PTP LAR is of special interest because it is localized at adherens junctions and at focal adhesions (this report and Kypta, *et al*. (1996) *J. Cell Biol*. **134**, 1519-1529; Serra-Pagès, *et al*. (1995) *EMBO J.* **14,** 2827-2838; Debant, *et al*. (1996) *Proc. Natl. Acad. Sci. USA* **93**, 5466-5471), thus PTP LAR could represent an essential regulator of the disassembly and reassembly of cell-cell- as well as cell-EGM-adhesions during epithelial cell migration.

We demonstrate, that modest ectopic overexpression of PTP LAR significantly inhibited epithelial cell migration. A similar situation is found in Drosophila, where PTP LAR, contrary to vertebrates, is almost exclusively expressed in developing neurons. In flies lacking PTP LAR, motor axons bypass their normal target region and instead continue to extend without stopping (Krueger, *et al*. (1996) *Cell* **84,** 611-622). PTP LAR, PTPµ, PTPκ as well as PTP DEP-1 were found to be expressed at elevated protein levels with increased cell confluence (Fuchs, *et al*. (1996) *J. Biol. Chem*. **271,** 16712-17719; Longo *et al*. (1993) *J. Biol. Chem*. **268,** 26503-26511; Gebbink, *et al*. (1995) *J. Cell Biol.* **131,** 251-260; Östman, *et al*. (1994) *Proc. Natl. Acad. Sci. USA* **91**, 9680-9684) thereby contributing to the observed increased phosphatase activity in confluent cells (Mansbridge, *et al*. (1992) *J. Cell Physiol*. **151**, 433-442).

The increase in phosphatase activity in confluent cells suggests a role of PTPs in the regulation and stabilization of cell-cell-contracts and epithelial cell integrity. In contrast, during wound healing, cells at the wound edge are in subconfluent situation where PTP action is reduced. This could favor tyrosine kinase signaling through stimulation by growth factors such as EGF, TGF α, and KGF, which results in an increase in cell migration and proliferation and thus to accelerated wound healing since these growth factors were increased during a wound situation (Buckley, *et al*. (1985) *Proc. Natl. Acad. Sci. USA* **82**; Rappolee, *et al*. (1988) *Science* **241**, 708-712; Werner, *et al*. (1992) *Proc. Natl. Acad*. *Sci. USA* **89**, 6896-6900).

The importance of free and tyrosine phosphorylated β-catenin during epithelial cell migration is underscored by our observations that interfering with this parameter by overexpressing hPTP LAR inhibits epithelial cell motility. Furthermore, expression of hPTP LAR in NBT II cells inhibited tumor formation in nude mice, although the growth characteristics of these cells *in vitro* were not altered. Ectopic expression of hPTP LAR to about twice the endogenous level was sufficient to result in the biological effects observed. Such modest ectopic expression of hPTP LAR appears to be critical, since high overexpression results in completely altered growth characteristics and apoptosis of the cells (Weng, *et al*. (1998) *Curr. Biol*. **8**, 247-256). The data presented in this report strongly support an important role for PTP LAR in the regulation of cell-cell adhesion and epithelial cell migration as well as tumorigenesis by controlling the free pool of signaling β-catenin.

Since mutations or deletions in either APC or β-catenin leading to a stabilized pool of free β-catenin have been correlated with tumor formation (Korinek, *et al*. (1997) *Science* **275,** 1784-178; Morin, *et al*. (1997) *Science* **275,** 1787-1790; Rubinfeld, *et al*. (1997) *Science* **275,** 1790-1792), loss of PTP LAR function may also contribute to tumor formation and metastasis. As a potential tumor suppressor gene product, PTP LAR could therefore serve as a prognostic marker for human cancer.

### I. The Nucleic Acids Sequence of PTP LAR

Included for the methods of use for this invention are the functional equivalents of the herein-described isolated nucleic acid molecules. The degeneracy of the genetic code permits substitution of certain codons by other codons that specify the same amino acid and hence would give rise to the same protein. The nucleic acid sequence can vary substantially since, with the exception of methionine and tryptophan, the known amino acids can be coded for by more than one codon. Thus, portions or all of the PTP LAR gene could be synthesized to give a nucleic acid sequence significantly different from that shown in Figure 10. The encoded amino acid sequence thereof would, however, be preserved.

In addition, the nucleic acid sequence may comprise a nucleotide sequence which results from the addition, deletion or substitution of at least one nucleotide to the 5'-end and/or the 3'-end of the nucleic acid formula shown in figure 10, or a derivative thereof. Any nucleotide or polynucleotide may be used in this regard, provided that its addition, deletion or substitution does not alter the amino acid sequence of PTP LAR, which is encoded by the nucleotide sequence. For example, the methods of the present invention are intended to include for their use any nucleic acid sequence resulting from the addition of ATG as an initiation codon at the 5'-end of the PTP LAR nucleic acid sequence or its derivative, or from the addition of TTA, TAG or TGA as a termination codon at the 3'-end of the PTP LAR nucleotide sequence or its derivative. Moreover, PTP LAR may, as necessary, have restriction endonuclease recognition sites added to its 5'-end and/or 3'-end.

Such functional alterations of a given nucleic acid sequence afford an opportunity to promote secretion and/or processing of heterologous proteins encoded by foreign nucleic acid sequences fused thereto. All variations of the nucleotide sequence of PTP LAR and fragments thereof permitted by the genetic code are, therefore, included in this invention.

Further, it is possible to delete codons or to substitute one or more codons with codons other than degenerate codons to produce a structurally modified polypeptide, but one which has substantially the same utility or activity as the polypeptide produced by the unmodified nucleic acid molecule. As recognized in the art, the two polypeptides are functionally equivalent, as are the two nucleic acid molecules that give rise to their production, even though the differences between the nucleic acid molecules are not related to the degeneracy of the genetic code. These nucleic acids are also intended to be used in the methods of the invention.

### II. Nucleic Acid Probes, Methods, and Kits for Detection of PTP LAR.

One skilled in the art can readily design probes based on the sequence disclosed herein using methods of computer alignment and sequence analysis known in the art (cf. "Molecular Cloning: A Laboratory Manual", second edition, Cold Spring Harbor Laboratory, Sambrook, Fritsch, & Maniatis, eds., 1989). The hybridization probes used in the methods of the present invention can be labeled by standard labeling techniques such as with a radiolabel, enzyme label, fluorescent label, biotin-avidin label, chemiluminescence, and the like. After hybridization, the probes may be visualized using known methods.

The nucleic acid probes to be used in the methods of the present invention include RNA, as well as DNA probes, such probes being generated using techniques known in the art. The nucleic acid probe may be immobilized on a solid support. Examples of such solid supports include, but are not limited to, plastics such as polycarbonate, complex carbohydrates such as agarose and sepharose, and acrylic resins, such as polyacrylamide and latex beads. Techniques for coupling nucleic acid probes to such solid supports are well known in the art.

The test samples suitable for nucleic acid probing methods of the present invention include, for example, cells or nucleic acid extracts of cells, or biological fluids. The samples used in the above-described methods will vary based on the assay format, the detection method and the nature of the tissues, cells or extracts to be assayed. Methods for preparing nucleic acid extracts of cells are well known in the art and can be readily adapted in order to obtain a sample that is compatible with the method utilized.

One method of detecting the presence of PTP LAR in a sample comprises (a) contacting said sample with the above-described nucleic acid probe under conditions such that hybridization occurs, and (b) detecting the presence of said probe bound to said nucleic acid molecule. One skilled in the art would select the nucleic acid probe according to techniques known in the art as described above. Samples to be tested include but should not be limited to RNA samples of human tissue.

A kit for detecting the presence of PTP LAR in a sample comprises at least one container means having disposed therein the above-described nucleic acid probe. The kit may further comprise other containers comprising one or more of the following: wash reagents and reagents capable of detecting the presence of bound nucleic acid probe. Examples of detection reagents include, but are not limited to radiolabelled probes, enzymatic labeled probes (horseradish peroxidase, alkaline phosphatase), and affinity labeled probes (biotin, avidin, or steptavidin).

In detail, a compartmentalized kit includes any kit in which reagents are contained in separate containers. Such containers include small glass containers, plastic containers or strips of plastic or paper. Such containers allow the efficient transfer of reagents from one compartment to another compartment such that the samples and reagents are not cross-contaminated and the agents or solutions of each container can be added in a quantitative fashion from one compartment to another. Such containers will include a container which will accept the test sample, a container which contains the probe or primers used in the assay, containers which contain wash reagents (such as phosphate buffered saline, Tris-buffers, and the like), and containers which contain the reagents used to detect the hybridized probe, bound antibody, amplified product, or the like. One skilled in the art will readily recognize that the nucleic acid probes described in the present invention can readily be incorporated into one of the established kit formats which are well known in the art.

### III. DNA Constructs Comprising a PTP LAR Nucleic Acid Molecule and Cells Containing These Constructs.

The present invention also relates to a recombinant DNA molecule comprising, 5' to 3', a promoter effective to initiate transcription in a host cell and the above-described nucleic acid molecules. In addition, the present invention relates to a recombinant DNA molecule comprising a vector and an above-described nucleic acid molecule. The present invention also relates to a nucleic acid molecule comprising a transcriptional region functional in a cell, a sequence complementary to an RNA sequence encoding an amino acid sequence corresponding to the above-described polypeptide, and a transcriptional termination region functional in said cell. The above-described molecules may be isolated and/or purified DNA molecules.

The present invention also relates to a cell or organism that contains an above-described nucleic acid molecule and thereby is capable of expressing a polypeptide. The polypeptide may be purified from cells that have been altered to express the polypeptide. A cell is said to be "altered to express a desired polypeptide" when the cell, through genetic manipulation, is made to produce a protein which it normally does not produce or which the cell normally produces at lower levels. One skilled in the art can readily adapt procedures for introducing and expressing either genomic, cDNA, or synthetic sequences into either eukaryotic or prokaryotic cells.

A nucleic acid molecule, such as DNA, is said to be "capable of expressing" a polypeptide if it contains nucleotide sequences which contain transcriptional and translational regulatory information and such sequences are "operably linked" to nucleotide sequences which encode the polypeptide. An operable linkage is a linkage in which the regulatory DNA sequences and the DNA sequence sought to be expressed are connected in such a way as to permit gene sequence expression. The precise nature of the regulatory regions needed for gene sequence expression may vary from organism to organism, but shall in general include a promoter region which, in prokaryotes, contains both the promoter (which directs the initiation of RNA transcription) as well as the DNA sequences which, when transcribed into RNA, will signal synthesis initiation. Such regions will normally include those 5'-non-coding sequences involved with initiation of transcription and translation, such as the TATA box, capping sequence, CAAT sequence, and the like.

Two DNA sequences (such as a promoter region sequence and a sequence encoding PTP LAR) are said to be operably linked if the nature of the linkage between the two DNA sequences does not (1) result in the introduction of a frame-shift mutation, (2) interfere with the ability of the promoter region sequence to direct the transcription of a gene sequence encoding a phosphatase of the invention, or (3) interfere with the ability of the gene sequence of PTP LAR to be transcribed by the promoter region sequence. Thus, a promoter region would be operably linked to a DNA sequence if the promoter were capable of effecting transcription of that DNA sequence. Thus, to express a gene encoding PTP LAR, transcriptional and translational signals recognized by an appropriate host are necessary.

The present invention encompasses the expression of PTP LAR (or a functional derivative thereof) in either prokaryotic or eukaryotic cells. Prokaryotic hosts are, generally, very efficient and convenient for the production of recombinant proteins and are, therefore, one type of preferred expression system for PTP LAR. Prokaryotes most frequently are represented by various strains of *E. coli.* However, other microbial strains may also be used, including other bacterial strains.

In prokaryotic systems, plasmid vectors that contain replication sites and control sequences derived from a species compatible with the host may be used. Examples of suitable plasmid vectors may include pBR322, pUC118, pUC119 and the like; suitable phage or bacteriophage vectors may include γgt10, γgt11 and the like; and suitable virus vectors may include pMAM-neo, pKRC and the like. Preferably, the selected vector of the present invention has the capacity to replicate in the selected host cell.

Recognized prokaryotic hosts include bacteria such as *E. coli, Bacillus, Streptomyces, Pseudomonas, Salmonella, Serratia*, and the like. However, under such conditions, the polypeptide will not be glycosylated. The prokaryotic host must be compatible with the replicon and control sequences in the expression plasmid.

To express PTP LAR (or a functional derivative thereof) in a prokaryotic cell, it is necessary to operably link the sequence encoding the kinase of the invention to a functional prokaryotic promoter. Such promoters may be either constitutive or, more preferably, regulatable (*i.e.*, inducible or derepressible). Examples of constitutive promoters include the *int* promoter of bacteriophage λ, the *bla* promoter of the β-lactamase gene sequence of pBR322, and the cat promoter of the chloramphenicol acetyl transferase gene sequence of pPR325, and the like. Examples of inducible prokaryotic promoters include the major right and left promoters of bacteriophage λ (P_{L} and P_{R}), the *trp*, *recA, λacZ*, λ*acI*, and *gal* promoters of *E. coli,* the α-amylase (Ulmanen *et al*., J. Bacteriol. 162:176-182, 1985) and the ζ-28-specific promoters of *B. subtilis* (Gilman *et al*., Gene Sequence 32:11-20, 1984), the promoters of the bacteriophages of *Bacillus* (Gryczan, In: The Molecular Biology of the Bacilli, Academic Press, Inc., NY, 1982), and *Streptomyces* promoters (Ward *et al*., Mol. Gen. Genet. 203:468-478, 1986). Prokaryotic promoters are reviewed by Glick (Ind. Microbiot. 1:277-282, 1987), Cenatiempo (Biochimie 68:505-516, 1986), and Gottesman (Ann. Rev. Genet. 18:415-442, 1984).

Proper expression in a prokaryotic cell also requires the presence of a ribosome-binding site upstream of the gene sequence-encoding sequence. Such ribosome-binding sites are disclosed, for example, by Gold *et al*. (Ann. Rev. Microbiol. 35:365-404, 1981). The selection of control sequences, expression vectors, transformation methods, and the like, are dependent on the type of host cell used to express the gene. As used herein, "cell", "cell line", and "cell culture" may be used interchangeably and all such designations include progeny. Thus, the words "transformants" or "transformed cells" include the primary subject cell and cultures derived therefrom, without regard to the number of transfers. It is also understood that all progeny may not be precisely identical in DNA content, due to deliberate or inadvertent mutations. However, as defined, mutant progeny have the same functionality as that of the originally transformed cell.

Host cells which may be used in the expression systems of the present invention are not strictly limited, provided that they are suitable for use in the expression of the kinase polypeptide of interest. Suitable hosts may often include eukaryotic cells. Preferred eukaryotic hosts include, for example, yeast, fungi, insect cells, mammalian cells either *in vivo*, or in tissue culture. Mammalian cells which may be useful as hosts include HeLa cells, cells of fibroblast origin such as VERO or CHO-K1, or cells of lymphoid origin and their derivatives. Preferred mammalian host cells include SP2/0 and J558L, as well as neuroblastoma cell lines such as IMR 332, which may provide better capacities for correct post-translational processing.

In addition, plant cells are also available as hosts, and control sequences compatible with plant cells are available, such as the cauliflower mosaic virus 35S and 19S, and nopaline synthase promoter and polyadenylation signal sequences. Another preferred host is an insect cell, for example the *Drosophila* larvae. Using insect cells as hosts, the *Drosophila* alcohol dehydrogenase promoter can be used (Rubin, Science 240:1453-1459, 1988). Alternatively, baculovirus vectors can be engineered to express large amounts of PTP LAR in insect cells (Jasny, Science 238:1653, 1987; Miller *et al*., In: Genetic Engineering, Vol. 8, Plenum, Setlow *et al*., eds., pp. 277-297, 1986).

Any of a series of yeast expression systems can be utilized which incorporate promoter and termination elements from the actively expressed sequences coding for glycolytic enzymes that are produced in large quantities when yeast are grown in mediums rich in glucose. Known glycolytic gene sequences can also provide very efficient transcriptional control signals. Yeast provides substantial advantages in that it can also carry out post-translational modifications. A number of recombinant DNA strategies exist utilizing strong promoter sequences and high copy number plasmids which can be utilized for production of the desired proteins in yeast. Yeast recognizes leader sequences on cloned mammalian genes and secretes peptides bearing leader sequences (*i.e.*, pre-peptides). Several possible vector systems are available for the expression of kinases of the invention in a mammalian host.

A wide variety of transcriptional and translational regulatory sequences may be employed, depending upon the nature of the host. The transcriptional and translational regulatory signals may be derived from viral sources, such as adenovirus, bovine papilloma virus, cytomegalovirus, simian virus, or the like, where the regulatory signals are associated with a particular gene sequence which has a high level of expression. Alternatively, promoters from mammalian expression products, such as actin, collagen, myosin, and the like, may be employed. Transcriptional initiation regulatory signals may be selected which allow for repression or activation, so that expression of the gene sequences can be modulated. Of interest are regulatory signals which are temperature-sensitive so that by varying the temperature, expression can be repressed or initiated, or are subject to chemical (such as metabolite) regulation.

Expression of PTP LAR in eukaryotic hosts requires the use of eukaryotic regulatory regions. Such regions will, in general, include a promoter region sufficient to direct the initiation of RNA synthesis. Preferred eukaryotic promoters include, for example, the promoter of the mouse metallothionein I gene sequence (Hamer *et al*., J. Mol. Appl. Gen. 1:273-288, 1982); the TK promoter of Herpes virus (McKnight, Cell 31:355-365, 1982); the SV40 early promoter (Benoist *et al*., Nature (London) 290:304-31, 1981); and the yeast gal4 gene sequence promoter (Johnston *et al*., Proc. Natl. Acad. Sci. (USA) 79:6971-6975, 1982; Silver *et al*., Proc. Natl. Acad. Sci. (USA) 81:5951-5955, 1984).

Translation of eukaryotic mRNA is initiated at the codon which encodes the first methionine. For this reason, it is preferable to ensure that the linkage between a eukaryotic promoter and a DNA sequence which encodes PTP LAR (or a functional derivative thereof) does not contain any intervening codons which are capable of encoding a methionine (*i.e.*, AUG). The presence of such codons results either in the formation of a fusion protein (if the AUG codon is in the same reading frame AS PTP LAR coding sequence) or a frame-shift mutation (if the AUG codon is not in the same reading frame as the PTP LAR coding sequence).

A nucleic acid molecule encoding PTP LAR and an operably linked promoter may be introduced into a recipient prokaryotic or eukaryotic cell either as a nonreplicating DNA or RNA molecule, which may either be a linear molecule or, more preferably, a closed covalent circular molecule. Since such molecules are incapable of autonomous replication, the expression of the gene may occur through the transient expression of the introduced sequence. Alternatively, permanent expression may occur through the integration of the introduced DNA sequence into the host chromosome.

A vector may be employed which is capable of integrating the desired gene sequences into the host cell chromosome. Cells which have stably integrated the introduced DNA into their chromosomes can be selected by also introducing one or more markers which allow for selection of host cells which contain the expression vector. The marker may provide for prototrophy to an auxotrophic host, biocide resistance, *e.g.*, antibiotics, or heavy metals, such as copper, or the like. The selectable marker gene sequence can either be directly linked to the DNA gene sequences to be expressed, or introduced into the same cell by co-transfection. Additional elements may also be needed for optimal synthesis of mRNA. These elements may include splice signals, as well as transcription promoters, enhancers, and termination signals. cDNA expression vectors incorporating such elements include those described by Okayama (Mol. Cell. Biol. 3:280-, 1983).

The introduced nucleic acid molecule can be incorporated into a plasmid or viral vector capable of autonomous replication in the recipient host. Any of a wide variety of vectors may be employed for this purpose. Factors of importance in selecting a particular plasmid or viral vector include: the ease with which recipient cells that contain the vector may be recognized and selected from those recipient cells which do not contain the vector; the number of copies of the vector which are desired in a particular host; and whether it is desirable to be able to "shuttle" the vector between host cells of different species.

Preferred prokaryotic vectors include plasmids such as those capable of replication in *E*. *coli* (such as, for example, pBR322, ColEl, pSC101, pACYC 184, πVX; "Molecular Cloning: A Laboratory Manual", 1989, supra). Bacillus plasmids include pC194, pC221, pT127, and the like (Gryczan, In: The Molecular Biology of the Bacilli, Academic Press, NY, pp. 307-329, 1982). Suitable *Streptomyces* plasmids include p1J101 (Kendall *et al*., J. Bacteriol. 169:4177-4183, 1987), and streptomyces bacteriophages such as φC31 (Chater *et al*., In: Sixth International Symposium on Actinomycetales Biology, Akademiai Kaido, Budapest, Hungary, pp. 45-54, 1986). *Pseudomonas* plasmids are reviewed by John *et al*. (Rev. Infect. Dis. 8:693-704, 1986), and Izaki (Jpn. J. Bacteriol. 33:729-742, 1978).

Preferred eukaryotic plasmids include, for example, BPV, vaccinia, SV40, 2-micron circle, and the like, or their derivatives. Such plasmids are well known in the art (Botstein *et al*., Miami Wntr. Symp. 19:265-274, 1982; Broach, In: "The Molecular Biology of the Yeast Saccharomyces: Life Cycle and Inheritance", Cold Spring Harbor Laboratory, Cold Spring Harbor, NY, p. 445-470, 1981; Broach, Cell 28:203-204, 1982; Bollon *et al*., J. Clin. Hematol. Oncol. 10:39-48, 1980; Maniatis, In: Cell Biology: A Comprehensive Treatise, Vol. 3, Gene Sequence Expression, Academic Press, NY, pp. 563-608, 1980).

Once the vector or nucleic acid molecule containing the construct(s) has been prepared for expression, the DNA construct(s) may be introduced into an appropriate host cell by any of a variety of suitable means, i.e., transformation, transfection, conjugation, protoplast fusion, electroporation, particle gun technology, calcium phosphate-precipitation, direct microinjection, and the like. After the introduction of the vector, recipient cells are grown in a selective medium, which selects for the growth of vector-containing cells. Expression of the cloned gene(s) results in the production of a kinase of the invention, or fragments thereof. This can take place in the transformed cells as such, or following the induction of these cells to differentiate (for example, by administration of bromodeoxyuracil to neuroblastoma cells or the like). A variety of incubation conditions can be used to form the peptide of the present invention. The most preferred conditions are those which mimic physiological conditions.

### IV. The PTP LAR Protein

A variety of methodologies known in the art can be utilized to obtain the PTP LAR polypeptide. The polypeptide may be purified from tissues or cells that naturally produce the polypeptides. Alternatively, the above-described isolated nucleic acid fragments could be used to express PTP LAR in any organism. The samples of the present invention include cells, protein extracts or membrane extracts of cells, or biological fluids. The samples will vary based on the assay format, the detection method, and the nature of the tissues, cells or extracts used as the sample.

Any eukaryotic organism can be used as a source for the PTP LAR polypeptide, as long as the source organism naturally contains such polypeptides. As used herein, "source organism" refers to the original organism from which the amino acid sequence of the subunit is derived, regardless of the organism the subunit is expressed in and ultimately isolated from.

One skilled in the art can readily follow known methods for isolating proteins in order to obtain the polypeptides free of natural contaminants. These include, but are not limited to: size-exclusion chromatography, HPLC, ion-exchange chromatography, and immuno-affinity chromatography.

### V. Antibodies, Hybridomas, Methods of Use and Kits for Detection of PTP LAR

The present invention relates to an antibody having binding affinity to PTP LAR or methods of using these antibodies. The polypeptide may have the amino acid sequence set forth in figure 9, or a functional derivative thereof, or at least 9 contiguous amino acids thereof (preferably, at least 20, 30, 35, or 40 or more contiguous amino acids thereof).

The present invention also relates to an antibody having specific binding affinity PTP LAR or uses thereof. Such an antibody may be isolated by comparing its binding affinity to PTP LAR with its binding affinity to other polypeptides. Those which bind selectively to PTP LAR would be chosen for use in methods requiring a distinction between PTP LAR and other polypeptides. Such methods could include, but should not be limited to, the analysis of altered PTP LAR expression in tissue containing other polypeptides.

PTP LAR can be used to produce antibodies or hybridomas. One skilled in the art will recognize that if an antibody is desired, such a peptide could be generated as described herein and used as an immunogen. The antibodies of the present invention include monoclonal and polyclonal antibodies, as well fragments of these antibodies, and humanized forms. Humanized forms of the antibodies of the present invention may be generated using one of the procedures known in the art such as chimerization or CDR grafting.

The present invention also relates to a hybridoma that produces the above-described monoclonal antibody, or binding fragment thereof. A hybridoma is an immortalized cell line that is capable of secreting a specific monoclonal antibody.

In general, techniques for preparing monoclonal antibodies and hybridomas are well known in the art (Campbell, "Monoclonal Antibody Technology: Laboratory Techniques in Biochemistry and Molecular Biology," Elsevier Science Publishers, Amsterdam, The Netherlands, 1984; St. *Groth et al*., J. Immunol. Methods 35:1-21, 1980). Any animal (mouse, rabbit, and the like) which is known to produce antibodies can be immunized with the selected polypeptide. Methods for immunization are well known in the art. Such methods include subcutaneous or intraperitoneal injection of the polypeptide. One skilled in the art will recognize that the amount of polypeptide used for immunization will vary based on the animal that is immunized, the antigenicity of the polypeptide and the site of injection.

The polypeptide may be modified or administered in an adjuvant in order to increase the peptide antigenicity. Methods of increasing the antigenicity of a polypeptide are well known in the art. Such procedures include coupling the antigen with a heterologous protein (such as globulin or β-galactosidase) or through the inclusion of an adjuvant during immunization.

For monoclonal antibodies, spleen cells from the immunized animals are removed, fused with myeloma cells, such as SP2/0-Agl4 myeloma cells, and allowed to become monoclonal antibody producing hybridoma cells. Any one of a number of methods well known in the art can be used to identify the hybridoma cell that produces an antibody with the desired characteristics. These include screening the hybridomas with an ELISA assay, western blot analysis, or radioimmunoassay (Lutz *et al*., Exp. Cell Res. 175:109-124, 1988). Hybridomas secreting the desired antibodies are cloned and the class and subclass are determined using procedures known in the art (Campbell, "Monoclonal Antibody Technology: Laboratory Techniques in Biochemistry and Molecular Biology", supra, 1984).

For polyclonal antibodies, antibody-containing antisera is isolated from the immunized animal and is screened for the presence of antibodies with the desired specificity using one of the above-described procedures. The above-described antibodies may be detectably labeled. Antibodies can be detectably labeled through the use of radioisotopes, affinity labels (such as biotin, avidin, and the like), enzymatic labels (such as horse radish peroxidase, alkaline phosphatase, and the like) fluorescent labels (such as FITC or rhodamine, and the like), paramagnetic atoms, and the like. Procedures for accomplishing such labeling are well-known in the art, for example, *see* Stemberger *et al*., J. Histochem. Cytochem. 18:315, 1970; Bayer *et al*., Meth. Enzym. 62:308-, 1979; Engval *et al*., Immunol. 109:129-, 1972; Goding, J. Immunol. Meth. 13:215-, 1976. The labeled antibodies of the present invention can be used for *in vitro, in vivo,* and *in situ* assays to identify cells or tissues that express a specific peptide.

The above-described antibodies may also be immobilized on a solid support. Examples of such solid supports include plastics such as polycarbonate, complex carbohydrates such as agarose and sepharose, acrylic resins and such as polyacrylamide and latex beads. Techniques for coupling antibodies to such solid supports are well known in the art (Weir *et al*., "Handbook of Experimental Immunology" 4th Ed., Blackwell Scientific Publications, Oxford, England, Chapter 10, 1986; Jacoby *et al*., Meth. Enzym. 34, Academic Press, N.Y., 1974). The immobilized antibodies of the present invention can be used for *in vitro*, *in vivo,* and *in situ* assays as well as in immunochromotography.

Furthermore, one skilled in the art can readily adapt currently available procedures, as well as the techniques, methods and kits disclosed herein with regard to antibodies, to generate peptides capable of binding to a specific peptide sequence in order to generate rationally designed antipeptide peptides (Hurby *et al*., "Application of Synthetic Peptides: Antisense Peptides", In Synthetic Peptides, A User's Guide, W.H. Freeman, NY, pp. 289-307, 1992; Kaspczak *et al*., Biochemistry 28:9230-9238, 1989).

Anti-peptide peptides can be generated by replacing the basic amino acid residues found in the peptide sequences of the kinases of the invention with acidic residues, while maintaining hydrophobic and uncharged polar groups. For example, lysine, arginine, and/or histidine residues are replaced with aspartic acid or glutamic acid and glutamic acid residues are replaced by lysine, arginine or histidine.

The present invention also relates to a method of detecting a PTP LAR in a sample, comprising: (a) contacting the sample with an above-described antibody, under conditions such that immunocomplexes form, and (b) detecting the presence of said antibody bound to the polypeptide. In detail, the methods comprise incubating a test sample with one or more of the antibodies of the present invention and assaying whether the antibody binds to the test sample. Altered levels of a PTP LAR of the invention in a sample as compared to normal levels may indicate disease or disease prognosis.

Conditions for incubating an antibody with a test sample vary. Incubation conditions depend on the format employed in the assay, the detection methods employed, and the type and nature of the antibody used in the assay. One skilled in the art will recognize that any one of the commonly available immunological assay formats (such as radioimmunoassays, enzyme-linked immunosorbent assays, diffusion based Ouchterlony, or rocket immunofluorescent assays) can readily be adapted to employ the antibodies of the present invention. Examples of such assays can be found in Chard ("An Introduction to Radioimmunoassay and Related Techniques" Elsevier Science Publishers, Amsterdam, The Netherlands, 1986), Bullock *et al*. ("Techniques in Immunocytochemistry," Academic Press, Orlando, FL Vol. 1, 1982; Vol. 2, 1983; Vol. 3, 1985), Tijssen ("Practice and Theory of Enzyme Immunoassays: Laboratory Techniques in Biochemistry and Molecular Biology," Elsevier Science Publishers, Amsterdam, The Netherlands, 1985).

The immunological assay test samples of the present invention include cells, protein or membrane extracts of cells, or biological fluids such as blood, serum, plasma, or urine. The test samples used in the above-described method will vary based on the assay format, nature of the detection method and the tissues, cells or extracts used as the sample to be assayed. Methods for preparing protein extracts or membrane extracts of cells are well known in the art and can be readily be adapted in order to obtain a sample which is testable with the system utilized.

A kit contains all the necessary reagents to carry out the previously described methods of detection. The kit may comprise: (i) a first container means containing an above-described antibody, and (ii) second container means containing a conjugate comprising a binding partner of the antibody and a label. In another preferred embodiment, the kit further comprises one or more other containers comprising one or more of the following: wash reagents and reagents capable of detecting the presence of bound antibodies.

Examples of detection reagents include, but are not limited to, labeled secondary antibodies, or in the alternative, if the primary antibody is labeled, the chromophoric, enzymatic, or antibody binding reagents which are capable of reacting with the labeled antibody. The compartmentalized kit may be as described above for nucleic acid probe kits. One skilled in the art will readily recognize that the antibodies described in the present invention can readily be incorporated into one of the established kit formats that are well known in the art.

### VI. Isolation of Compounds Which Interact With PTP LAR

The present invention also relates to a method of detecting a compound capable of binding to PTP LAR comprising incubating the compound with a PTP LAR and detecting the presence of the compound bound to PTP LAR. The compound may be present within a complex mixture, for example, serum, body fluid, or cell extracts.

The present invention also relates to a method of detecting an agonist or antagonist of PTP LAR activity or PTP LAR binding partner activity comprising incubating cells that produce a PTP LAR in the presence of a compound and detecting changes in the level of PTP LAR activity or PTP LAR binding partner activity. The compounds thus identified would produce a change in activity indicative of the presence of the compound. The compound may be present within a complex mixture, for example, serum, body fluid, or cell extracts. Once the compound is identified it can be isolated using techniques well known in the art.

In an effort to discover novel treatments for diseases, biomedical researchers and chemists have designed, synthesized, and tested molecules that inhibit the function of many proteins. Some small organic molecules form a class of compounds that modulate the function of proteins. Examples of molecules that have been reported to inhibit the function of proteins, for example, include, but are not limited to, bis monocyclic, bicyclic or heterocyclic aryl compounds (PCT WO 92/20642, published November 26, 1992 by Maguire *et al*.), vinylene-azaindole derivatives (PCT WO 94/14808, published July 7, 1994 by Ballinari *et al*.), 1-cyclopropyl-4-pyridyl-quinolones (U.S. Patent No. 5,330,992), styryl compounds (U.S. Patent No. 5,217,999), styryl-substituted pyridyl compounds (U.S. Patent No. 5,302,606), certain quinazoline derivatives (EP Application No. 0 566 266 A1), seleoindoles and selenides (PCT WO 94/03427, published February 17, 1994 by Denny *et al*.), tricyclic polyhydroxylic compounds (PCT WO 92/21660, published December 10, 1992 by Dow), and benzylphosphonic acid compounds (PCT WO 91/15495, published October 17, 1991 by Dow *et al*).

Compounds that can traverse cell membranes and are resistant to acid hydrolysis are potentially advantageous as therapeutics as they can become highly bioavailable after being administered orally to patients. However, many of these protein inhibitors only weakly inhibit the function of proteins. In addition, many inhibit a variety of proteins and will cause multiple side-effects as therapeutics for diseases.

Some indolinone compounds, however, form classes of acid resistant and membrane permeable organic molecules. WO 96/22976 (published August 1, 1996 by Ballinari *et al*.) describes hydrosoluble indolinone compounds that harbor tetralin, naphthalene, quinoline, and indole substituents fused to the oxindole ring. These bicyclic substituents are in turn substituted with polar moieties including hydroxylated alkyl, phosphate, and ether moieties. U.S. Patent Application Serial Nos. 08/702,232, filed August 23, 1996, entitled "Indolinone Combinatorial Libraries and Related Products and Methods for the Treatment of Disease" by Tang *et al*. (Lyon & Lyon Docket No. 221/187) and 08/485,323, filed June 7, 1995, entitled "Benzylidene-Z-Indoline Compounds for the Treatment of Disease" by Tang *et al*. (Lyon & Lyon Docket No. 223/298) and International Patent Publication WO 96/22976, published August 1, 1996 by Ballinari *et al*., all of which are incorporated herein by reference in their entirety, including any drawings, describe indolinone chemical libraries of indolinone compounds harboring other bicyclic moieties as well as monocyclic moieties fused to the oxindole ring. Applications 08/702,232, filed August 23, 1996, entitled "Indolinone Combinatorial Libraries and Related Products and Methods for the Treatment of Disease" by Tang *et al*. (Lyon & Lyon Docket No. 221/187), 08/485,323, filed June 7, 1995, entitled "Benzylidene-Z-Indoline Compounds for the Treatment of Disease" by Tang *et al*. (Lyon & Lyon Docket No. 223/298), and WO 96/22976, published August 1, 1996 by Ballinari *et al*. teach methods of indolinone synthesis, methods of testing the biological activity of indolinone compounds in cells, and inhibition patterns of indolinone derivatives.

Other examples of substances capable of modulating protein activity include, but are not limited to, tyrphostins, quinazolines, quinoxolines, and quinolines. The quinazolines, tyrphostins, quinolines, and quinoxolines referred to above include well known compounds such as those described in the literature. For example, representative publications describing quinazolines include Barker et al., EPO Publication No. 0 520 722 A1; Jones et al., U.S. Patent No.4,447,608; Kabbe et al., U.S. Patent No. 4,757,072; Kaul and Vougioukas, U.S. Patent No. 5, 316,553; Kreighbaum and Comer, U.S. Patent No. 4,343,940; Pegg and Wardleworth, EPO Publication No. 0 562 734 A1; Barker et al., Proc. of Am. Assoc. for Cancer Research 32:327 (1991); Bertino, J.R., Cancer Research 3:293-304 (1979); Bertino, J.R., Cancer Research 9(2 part 1):293-304 (1979); Curtin et al., Br. J. Cancer 53:361-368 (1986); Fernandes et al., Cancer Research 43:1117-1123 (1983); Ferris et al. J. Orct. Chem. 44(2):173-178; Fry et al., Science 265:1093-1095 (1994); Jackman et al., Cancer Research 51:5579-5586 (1981); Jones et al. J. Med. Chem. 29(6):1114-1118; Lee and Skibo, Biochemistry 26(23):7355-7362 (1987); Lemus et al., J. Org. Chem. 54:3511-3518 (1989); Ley and Seng, Synthesis 1975:415-522 (1975); Maxwell et al., Magnetic Resonance in Medicine 17:189-196 (1991); Mini et al., Cancer Research 45:325-330 (1985); Phillips and Castle, J. Heterocyclic Chem. 17(19):1489-1596 (1980); Reece et al., Cancer Research 47(11):2996-2999 (1977); Sculier et al., Cancer Immunol. and Immunother. 23:A65 (1986); Sikora et al., Cancer Letters 23:289-295 (1984); Sikora et al., Analytical Biochem. 172:344-355 (1988).

Quinoxaline is described in Kaul and Vougioukas, U.S. Patent No. 5,316,553, incorporated herein by reference in its entirety, including any drawings.

Quinolines are described in Dolle et al., J. Med. Chem. 37:2627-2629 (1994); MaGuire, J. Med. Chem. 37:2129-2131 (1994); Burke et al., J. Med. Chem. 36:425-432 (1993); and Burke et al. BioOrganic Med. Chem. Letters 2:1771-1774 (1992), all of which are incorporated by reference in their entirety, including any drawings.

Tyrphostins are described in Allen et al., Clin. Exp. Immunol. 91:141-156 (1993); Anafi et al., Blood 82:12:3524-3529 (1993); Baker et al., J. Cell Sci. 102:543-555 (1992); Bilder et al., Amer. Physiol. Soc. pp. 6363-6143:C721-C730 (1991); Brunton et al., Proceedings of Amer. Assoc. Cancer Rsch. 33:558 (1992); Bryckaert et al., Experimental Cell Research 199:255-261 (1992); Dong et al., J. Leukocyte Biology 53:53-60 (1993); Dong et al., J. Immunol. 151(5):2717-2724 (1993); Gazit et al., J. Med. Chem. 32:2344-2352 (1989); Gazit et al., " J. Med. Chem. 36:3556-3564 (1993); Kaur et al., Anti-Cancer Drugs 5:213-222 (1994); Kaur et al., King et al., Biochem. J. 275:413-418 (1991); Kuo et al., Cancer Letters 74:197-202 (1993); Levitzki, A., The FASEB J. 6:3275-3282 (1992); Lyall et al., J. Biol. Chem. 264:14503-14509 (1989); Peterson et al., The Prostate 22:335-345 (1993); Pillemer et al., Int. J. Cancer 50:80-85 (1992); Posner et al., Molecular Pharmacology 45:673-683 (1993); Rendu et al., Biol. Pharmacology 44(5):881-888 (1992); Sauro and Thomas, Life Sciences 53:371-376 (1993); Sauro and Thomas, J. Pharm. and Experimental Therapeutics 267(3):119-1125 (1993); Wolbring et al., J. Biol. Chem. 269(36):22470-22472 (1994); and Yoneda et al., Cancer Research 51:4430-4435 (1991).

Other compounds that could be used as modulators include oxindolinones such as those described in U.S. patent application Serial No. 08/702,232 filed August 23, 1996.

### EXAMPLES

The examples below are not limiting and are merely representative of various aspects and features of the present invention. The examples below demonstrate the role of PTP LAR in epithelial cell migration and in tumor inhibition.

### General Materials and Methods

*Cloning of α-catenin*, *β-catenin*, *and plakoglobin* (γ-*catenin)-* Human α-catenin (Accession number D14705), β-catenin (Z19054), and Plakoglobin (M23410) were amplified from cDNA generated from MCF7 cells by PCR. PCR products were cloned in an eukaryotic expression vector under the control of the CMV promoter and confirmed by sequence analysis.

*Cell Lines and Cell Culture-* All cell lines were obtained from the American Tissue Culture Collection. NBT II cells (CRL-1655) were grown in MFM supplemented with 1% non-essential-amino-acids, Earle's balanced salt, 2 mM glutamine, 1 mM sodium pyruvate and 10% FCS (Sigma). MCF7 cells (HTB22) were grown in RPMI medium supplemented with 2 mM glutamine and 10% FCS, human embryonic 293 kidney cells (CRL 1573) in Dulbecco's modified Eagle's medium supplemented with 1 mg/mL glucose, 2 mM glutamine and 10% FCS. FCS was routinely heat-inactivated. All media and supplements were obtained from Gibco BRL.

*Migration Assays-* For *in vitro* wound assays, NBT II cells were plated at a density of 7x10⁴ cells/cm². After 24 hours the confluent monolayer was scratched with a pipette tip to create a cell free area, growth factors were added, and wound closure was documented by photography. For scatter assays, cells were plated at a cell density of 1x10⁴ cells/cm². Growth factors were added after 24 hours, and the morphology of cells and the dispersion of small colonies were documented by photography. Quantification of migration was performed by counting single cells with fibroblastoid migration morphology compared to cells in groups with epithelial morphology in different, randomly chosen microscopic fields. 1000 cells per dish were counted. All migration assays were performed in triplicate. Tyrosine kinases (TK), mitogen-activated protein kinase (MAPK) or phosphatidylinositol-3-kinase (P13K), were inhibited by Genistein (250 µM; Biomol), added one hour before addition of the growth factors. As a control, the solvent DMSO was used. DNA synthesis was inhibited by the specific inhibitor of DNA polymerase, α Aphidicolin (1,5 µM; Serva) 1 hour before adding the growth factors.

*Tumor formation in Nude Mice-* NBT II cells were resuspended at a cell density of 2x10⁶/50 µL in MEM and injected subcutaneously into the flank region of Swiss nude mice (IGR Villejuif, Paris, France). Tumor formation was monitored by measuring the width (W) and length (L) of the tumors with W<L. The tumor volume was calculated according to the formula (W² x L x π/6). Assays were performed at least in triplicate.

*Generation of Recombinant Retroviruses and Retrovirus-mediated Gene Transfer-* Full-length PTP LAR (H. Saito, Harvard Medical School, Boston, USA) was subcloned into pLXSN vector (Miller, *et al*. (1989) *BioTechniques* **7,** hereby incorporated herein by reference in its entirety, including any drawings, figures, or tables). Stable NBT II cells lines were generated by retroviral gene transfer as described (Pear, *et al*. (1993) *Proc. Natl. Acad. Sci. USA* **90,** 8392-8396, hereby incorporated herein by reference in its entirety, including any drawings, figures, or tables). Polyclonal and clonal cell lines were selected in medium containing 0.5 mg/mL G418 (Gibco BRL, FRG). Ectopic expression was confirmed by immunoprecipitation and Western blot analysis.

*Transient Expression, Cell Lysis, and Immunoprecipitation-* Transient transfection of human 293 embryonic kidney cells was performed as described (Chen, *et al*. (1987) *Mol. Cell. Biol.* **7,** 2745-2752, hereby incorporated herein by reference in its entirety, including any drawings, figures, or tables). For biochemical analysis, NBT II cells were plated at a density of 1x10⁴ cells/cm² at the day before lysis. When indicated, cells were pretreated prior to lysis with Na-orthovanadate (1 mM) for the indicated period of time. After washing with ice cold PBS, cells were lysed in ice cold lysis buffer (50 mM HEPES, pH 7.5, 150 mM NaCl, 1 mM EDTA, 10% glycerol, 20 mM pyrophosphate, 1% Triton X-100, 100 mM NaFl, 2 mM phenylmethylsulfonyl fluoride, 20 µg/mL aprotinin, 20 µg/mL leupeptin, 0.7 µg/mL pepstatin, 0.2 mM ammonium-molybdate, 2 mM Na-orthovanadate) and precleared by centrifugation at 12,500 x g for 10 min. at 4°C. The protein concentrations of the supernatants were adjusted to be equal. HNTG buffer (20 mM HEPES, pH 7.5, 150 mM NaCl, 10 mM pyrophosphate, 10 mM NaF1, 0.2 mM ammonium-molybdate, 10% glycerol, 0.1% Triton X-100, 2 mM Na-orthovanadate) was added in a 1:1 ratio and immunoprecipitations were performed for 2 hours at 4°C. Protein A- or Protein G-Sepharose was added for additional 2 hours. Precipitates were washed three times with HNTG buffer and beads resuspended in SDS-sample buffer. For subsequent Western blot analysis, proteins separated by SDS-PAGE were transferred to nitrocellulose (Schleicher and Schuell) and incubated with the respective antibody. Proteins were visualized by the ECL system (Amersham). Before reprobing, blots were stripped by incubation for 1 hour in 68 mM Tris-HCl, pH 6.8, 2% SDS, and 0.1% β-mercaptoethanol at 50°C.

*In Vitro Binding Assays-* The plasmid coding for the GST-hPTP LARᵢ-fusion protein was constructed by PCR-amplification of the cDNA sequence between amino acids 1259-1881 of human PTP LAR and cloned into the appropriate pGEX vector (Pharmacia Biotech). *In vitro* mutagenesis (Kunkel, *et al*. (1987) *Methods Enzymol*. **154**, 367-382, hereby incorporated by reference herein in its entirety, including any drawings, figures, or tables) of the original vector pSP65-LAR yielded PTP LAR with either inactivated PTP domain 1, PTP LAR D₁ C1522S, or inactivated PTP domain 2, PTP LAR D₂ C1813S, which were also amplified by PCR between amino acids 1259-1881 of human PTP LAR and subcloned into pGEX vector. The integrity of the subcloned PCR products was confirmed by sequence analysis. GST-fusion proteins were expressed in *E. coli* and purified as described (Smith, *et al*. (1988) *Gene* **67**, 31-40, hereby incorporated by reference herein in its entirety, including any drawings, figures, or tables). 3 µg of GST-hPTP LAR₁-fusion protein, or a three-fold molar excess of GST, were incubated at 4°C with equal amounts of cell lysates, were immobilized on glutathione-sepharose (Sigma, FRG), and were washed three times with HNTG. Bound proteins were separated by SDS-PAGE for Western blotting.

*Affinity Precipitation-* The plasmid coding for the GST-E-cadherin cytoplasmic-fusion protein was constructed by amplification of the cDNA sequence between amino acids 734-884 of murine E-cadherin (Nagafuchi, *et al*. (1987) *Nature* **329**, 341-343,hereby incorporated by reference herein in its entirety, including any drawings, figures, or tables) by PCR and cloned into the appropriate pGEX vector. For affinity precipitation equal concentrations of precleared NBT II cell lysates were incubated with 5 µg of purified GST-E cadherin cytoplasmic-fusion protein or a three-fold molar excess of GST and immobilized on glutathione-sepharose. The resulting complexes were washed three times with HNTG and bound proteins separated by SDS-PAGE for Western blotting.

*Antisera*- Monoclonal antibody against phosphotyrosine (4G10) was obtained from UBI. α-catenin, β-catenin, Plakoglobin, and E-cadherin antibodies were obtained from Transduction Laboratories. A second antibody against E-cadherin was raised against a GST-fusion protein containing amino acids 834-913 of human E-cadherin. Monoclonal antibody 11.1A (M. Streuli, Dana Farber Cancer Institute, Boston, USA) recognizes the extracellular domain of human PTP LAR (Streuli, *et al*. (1992) *EMBO J*. **11,** 897-907). Rabbit antiserum #320 (Y. Schlessinger, New York University Medical Center, New York, USA) is directed against a peptide corresponding to the C-terminus of PTP LAR (amino acids 1868-1881).

*In Vitro Dephosphorylation Assay-* β-catenin was transiently overexpressed in human 293 embryonic kidney cells. Cells were serum-starved, treated with pervanadate (0.3 µM H₂O₂/ 0.1 mM Na-orthovanadate) for 10 minutes and lysed. Immunoprecipitations were performed as described above except that HNTG without pyrophosphate, ammonium-molybdate, and Na-orthovanadate was used. PTP activity towards tyrosine phosphorylated β-catenin was assayed in 200 µL reactions at 25°C containing 25 mM HEPES, pH 7.5, 5 mM EDTA, 10 mM DTT, and 1 mg/mL BSA with 200 ng of GST-hPTP LAR cytoplasmic, GST-hPTP LAR D₁ C1522S, or GST-hPTP LAR D₂ C1813S added. Reactions were stopped by washing with HNTG and subsequently separated by SDS-PAGE. The tyrosine phosphorylation status was analyzed by Western blotting using anti-phosphotyrosine antibody.

*Immunofluorescence-* NB II cells were plated at a density of 1x10⁴ cells/cm² or 7x10⁴ cells/cm². 24 hours later cells were fixed with 2% paraformaldehyde in phosphate buffered saline (PBS, pH 7.4, 125 mM sucrose). Autofluorescence was quenched with PBS glycine (100 mM glycine, 0.1% borhydrate in PGS) and the cells were permeabilized with 0.5% saponin in PBS (5 min.). Nonspecific antibody binding was blocked for 1 h with phosphate buffered gelatine (PBG: PBS, 0.5% bovine serum albumin, 0.045% cold water fish gelatine, 5% donkey serum). Primary antibody incubation was performed at room temperature for 2 h after dilution in PBG. After three washes in PBG primary antibody binding was detected with isotype specific secondary antibody, either FITC(DTAF)- or Cy3-conjugated (Jackson Laboratories, USA). For double labeling experiments antibody decoration was performed consecutively. Coverslips were mounted under Permafluor mounting medium (Immunotech, France) and viewed either with a conventional fluorescence microscope (Leica, FRG) or with a CLSM laser confocal microscope (Leica, FRG). Controls were recorded at identical settings.

### EXAMPLE 1: Relocalisation of the cadherin/catenin-complex upon induction of epithelial cell migration

Migration of epithelial cells in tissue culture represents in many aspects a model system of the epithelial-mesenchymal transition (Manske, *et al*. (1994) *Int. Rev. Cytol.* **155,** 49-9). The rat bladder carcinoma cell line NBT II (Toyoshima, *et al*. (1971) *J. Nat. Cancer Inst*. **47**, 979-985) was used, since certain growth factors and components of the extracellular matrix (ECM) induce migration of these cells (Valles, *et al*. (1990) *Proc. Natl*. *Acad. Sci. USA* **87**, 1124-1128; Tchao, R. (1982) *Cell Motil*. **4**, 333-341; Tucker, *et al*. (1990) *Cancer Res.* **50**, 129-137).

Results of an *in vitro* wound assay are shown in Figure 1A for EGF and the commercial serum substitute Ultroser G. To rule out proliferation as the cause of wound closure, Aphidicolin was used to inhibit DNA-synthesis; it did not interfere with migration. In contrast, inhibition of phosphatidylinositol-3-kinase (P13K) by 1y295002, tyrosine kinases (tk) by Genistein, or mitogen-activated protein kinases (MAPK) by PD98059, completely abolished cell migration. The protein levels of the components of the cadherin/catenin-complex remained unchanged during migration and even overexpression of E-cadherin could not prevent disruption of intercellular contacts (Boyer, *et al*. (1992) *Exp. Cell Res.* **201**, 347-357).

However, the localization of members of the cadherin/catenin-complex during EMT was altered. E-cadherin (green fluorescence) and β-catenin (red fluorescence) were located along the entire cell-cell contact region of adjacent, subconfluently plated cells (Fig. 1B upper panel). Fluorescence on contact-free membrane portions was only weak or absent. Some weak β-catenin staining was detected in the nucleus of control cells, most probably due to the fact that these subconfluent cells did not establish their adherens junctions completely. However, upon induction of migration by EGF, E-cadherin and β-catenin redistributed over the entire cell surface and into the cytoplasm. Interestingly, increased nuclear localization of β-catenin was also detectable during epithelial cell migration.

### EXAMPLE 2: Tyrosine phosphorylation of the cadherin/catenin-complex

The specificity of tyrosine kinases for the cadherin/catenin-complex was investigated. Transient cotransfection of TKs with members of the cadherin/catenin-complex demonstrated that only TKs that play a role in epithelial cell migration, such as EGFR, c-Src, or FGFR2, are capable of phosphorylating β-catenin and plakoglobin. However, α-catenin and E-cadherin were not substrates of these TKs. These observations could be confirmed in non-transfected NBT II cells stimulated to migrate. After cell lysis all members of the cadherin/catenin complex were immunoprecipitated with specific antibodies. This technique allows to precipitate the members of the cadherin/catenin complex regardless their localization and binding partners therefore also E-cadherin bound β-catenin. Tyrosine phosphorylation levels were analyzed by Western blotting with an anti-phosphotyrosine antibody (Fig. 2, top) followed by the detection of E-cadherin (Fig. 2, middle) or the catenins (Fig. 2, bottom) by specific antibodies.

Tyrosine phosphorylation of β-catenin and plakoglobin, but not of α-catenin and E-cadherin was already detectable 30 minutes after induction of migration. The phosphorylation was transient, lasted for about 9 hours, and was no longer detectable after 24 hours. For immunoprecipitation, pretreatment of the cells with 1mM Na-orthovanadate, a specific inhibitor of PTPs, was necessary to detect tyrosine phosphorylation. This indicates that the phosphorylation state of β-catenin and plakoglobin was tightly regulated by TKs and PTPs. The correlation of epithelial cell colony dispersion with tyrosine phosphorylation of β-catenin and plakoglobin could also be demonstrated in HT29- and HaCaT-cells, suggesting a common regulatory mechanism during the induction of cell migration.

### EXAMPLE 3: Increased free pool of β-catenin during epithelial cell migration

β-catenin from EGF-treated cells was affinity precipitated with a GST fusion protein comprising the entire cytoplasmic domain of E-cadherin. The levels of free, uncomplexed β-catenin (Fig. 3, top), as well as its phosphotyrosine content (Fig. 3, bottom), were analyzed by immunoblotting. As previously demonstrated, this strategy allows, in contrast to immunoprecipitation, the specific and selective precipitation of the free, non-E-cadherin-bound pool of β-catenin (Papkoff, *et al*. (1996) *Mol. Cell. Biol*. **16**, 2128-2134, hereby incorporated herein by reference in its entirety, including any drawings, figures, or tables).

The induction of migration by EGF correlated with an increase of the free pool of β-catenin which had a significantly higher phosphotyrosine content than β-catenin obtained by immunoprecipitation (compare Figures 2 and 3). The free pool of β-catenin in non-stimulated control cells was unaffected. While increases in free, tyrosine phosphorylated β-catenin were detected even without adding Na-orthovanadate (see Figure 8), its inclusion improved detection. These findings indicate that phosphorylation by TKs leads to an increase of the free, uncomplexed pool of β-catenin during epithelial cell migration.

### EXAMPLE 4: Colocalization of the cadherin/catenin-complex with PTP LAR

Pretreatment with Na-orthovanadate led to an increased tyrosine phosphorylation of β-catenin and plakoglobin in migrating NBT II cells. PTPs may therefore act as steady state equilibrium antagonists of TKs for regulatory events at adherens junctions. As shown in Figure 4, PTP LAR (red fluorescence) colocalized with the cadherin/catenin complex (green fluorescence) as indicated by the yellow fluorescence signal at adherens junctions of epithelial cells. PTP LAR has been localized to focal adhesions of epithelial MCF7 cells (Serra-Pagès, *et al*. (1995) *EMBO J.* **14**, 2827-2838). In NBT II cells, PTP LAR was detected predominantly at adherens junctions, and with a lower signal intensity at focal adhesions. Since motile cells have to rapidly disassemble and reassemble adherens junctions and focal adhesions, the localization of PTP LAR supports its potential regulatory function during epithelial cell migration.

### Example 5: Association of β-catenin and plakoglobin with PTP LAR

To investigate the potential association of PTP LAR with the cadherin/catenin-complex in intact cells, subconfluent human MCF7 cells were stimulated with EGF, were lysed, and were immunoprecipitated with antibodies either against PTP LAR or the cadherin/catenin-complex. Western blotting analysis with specific antibodies to β-catenin and E-cadherin (Fig.5A) or plakoglobin and E-cadherin (Fig.5B) detected these proteins in anti-human PTP LAR immunoprecipitates with the monoclonal antibody 11.1A against the extracellular domain of human PTP LAR and vice versa. Interestingly, the association was constitutive and independent of EGF stimulation. No specific signal of members of the cadherin/catenin-complex was obtained with the rabbit polyclonal antiserum #320 to PTP LAR, suggesting that the antigenic epitope was inaccessible in the complex. To investigate which component of the cadherin/catenin-complex mediated the interaction with PTP LAR, a GST fusion protein comprising the entire cytoplasmic domain of PTP LAR (GST-PTP LARᵢ) was used to affinity purify the individual components of the cadherin/catenin-complex, transiently overexpressed in human 293 embryonic kidney fibroblasts that had been treated with the tyrosine phosphatase inhibitor pervanadate before lysis.

β-catenin (Fig.5C, left) and plakoglobin (Fig.5C, right) were found to associate specifically with the cytoplasmic domain of PTP LAR under these conditions. E-cadherin and α-cadherin did not interact with PTP LAR directly under the same experimental conditions (data not shown). The phosphorylation state of β-catenin or plakoglobin, which were both tyrosine phosphorylated after treatment with pervanadate, did not affect their association with the GST-PTP LAR cytoplasmic fusion protein. This interaction required the complete cytoplasmic domain of PTP LAR since deletion mutants of PTP LAR did not bind β-catenin or plakoglobin efficiently.

### EXAMPLE 6: β-catenin is a substrate of PTP LAR in vitro

The association of β-catenin and plakoglobin with PTP LAR prompted the investigation of whether these proteins could represent actual substrates for PTP LAR. GST fusion proteins of PTP LAR comprising either the entire cytoplasmic domain (GST-PTP LAR cytopl.) or mutants of PTP LAR with catalytically inactivated PTP domain 1 (GST-PTP LAR PTP D₁ C1522S) or inactivated PTP domain 2 (GST-PTP LAR PTP D₂ C1813S) were incubated with tyrosine phosphorylated β-catenin from transiently overexpressing, pervanadate treated human 293 cells. After different time intervals, the reactions were terminated and analyzed with an anti-phosphotyrosine specific antibody.

A significant reduction in the tyrosine phosphorylation signal within the first five minutes after incubation of β-catenin with GST-PTP LAR cytoplasmic or GST-PTP LAR PTP D₂ C1813S (Fig.6, top, left and right) was revealed. No change in phosphorylation levels was observed after incubation with GST-PTP LAR PTP D₁ C1522S (Fig.6, top, middle), confirming results of enzymatic activity measurements of the GST fusion proteins using pNPP as a substrate (data not shown). These data are consistent with previous findings that the first PTP domain of PTP LAR is essential for catalytic activity whereas the second is catalytically inactive (Streuli, *et al*. (1990) *EMBO J.* **9**). Blots were reprobed with an anti-β-catenin specific antibody to confirm that equal amounts of protein were used in the assay (Fig.6, bottom). Thus, β-catenin is a substrate for PTP LAR.

### Example 7: PTP LAR inhibits epithelial cell migration and tumor formation in nude mice

We next examined whether PTP LAR has a direct regulatory function during epithelial cell migration. We therefore ectopically expressed human PTP LAR in NBT II cells at levels comparable to the endogenous protein thereby yielding polyclonal as well as clonal cell lines with about twice the PTP LAR expression relative to the parental cells. With these cell lines we performed scatter assays to quantify migration after EGF treatment. This modest ectopic expression of nPTP LAR in NBTII cells significantly inhibited EMT and motility (Fig.7A) to about 40% of the vector-control infected cell lines (NBT II pLXSN, Fig. 7B) without affecting the kinetics of the onset of migration. No differences in activation and autophosphorylation of the EGF receptor and its association with the adapter protein Shc were detected, suggesting that ectopic hPTP LAR does not function by inactivating the EGF-receptor. Furthermore, downstream events of EGF signaling like DNA-synthesis and proliferation rate of these NBT II-hPTP LAR cell lines were not affected by ectopic expression of hPTP LAR.

The epithelial cell migration in vitro resembles a simplified model system of tumor formation and metastasis *in vivo*. Therefore, the correlation of ectopic expression of hPTP LAR with a decreased capability of these cells to form tumors in vivo in nude mice was tested. NBT II hPTP LAR cells were injected subcutaneously into the flank region of Swiss nude mice. Interestingly, these cells displayed significantly reduced tumor growth in comparison to NBT II pLXSN cells (Fig. 7C). The capability to form tumors of polyclonal and clonal cell lines did not differ. The parental NBT II cells form tumors to the same extent as the vector-control infected cell lines which rules out a clonal artifact. These data strongly suggest that PTP LAR serves as a negative regulator of epithelial cell migration and tumor formation.

### EXAMPLE 8: PTP LAR inhibits tyrosine phosphorylation and the increase of the free pool of β-catenin

β-catenin was affinity precipitated in control and hPTP LAR-expressing NBT II cells using a GST-E-cadherin cytoplasmic fusion protein. The cells were not treated with the inhibitor of PTPs, Na-orthovanadate, so as to not mask the function of PTP LAR. Nevertheless, an increase in the free, uncomplexed and tyrosine phosphorylated pool of β-catenin in vector-infected control cells was detected, demonstrating that an increase in free, tyrosine phosphorylated β-catenin occurred also in cells without N-orthovanadate pretreatment (Fig. 8, left). However, in NBT II-hPTP LAR-expressing cells, neither tyrosine phosphorylation, nor an increase in the free pool of β-catenin were detectable (Fig. 8, right). Since β-catenin is a substrate of PTP LAR *in vitro* and ectopic expression of human PTP LAR in NBT II cells does not interfere with EGF receptor mediated signaling, PTP LAR functions as a specific negative regulator of β-catenin tyrosine phosphorylation, that prevents an increase in free β-catenin thereby inhibiting epithelial cell migration.

One skilled in the art would readily appreciate that the present invention is well adapted to carry out the objects and obtain the ends and advantages mentioned, as well as those inherent therein. The molecular complexes and the methods, procedures, molecules, specific compounds described herein are presently representative of preferred embodiments are exemplary and are not intended as limitations on the scope of the invention. Changes therein and other uses will occur to those skilled in the art which are encompassed within the spirit of the invention are defined by the scope of the claims.

It will be readily apparent to one skilled in the art that varying substitutions and modifications may be made to the invention disclosed herein without departing from the scope of the invention.

All patents and publications mentioned in the specification are indicative of the levels of those skilled in the art to which the invention pertains.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed.

In addition, where features or aspects of the invention are described in terms of Markush groups, those skilled in the art will recognize that the invention is also thereby described in terms of any individual member or subgroup of members of the Markush group. For example, if X is described as selected from the group consisting of bromine, chlorine, and iodine, claims for X being bromine and claims for X being bromine and chlorine are fully described.

Other embodiments are within the following claims.

## Claims

1. A method for prognosis of a disease or disorder by detection of PTP LAR in a sample,
wherein said sample is selected from the group consisting of cells, nucleic acid extracts of cells and biological fluids,
wherein said disease or disorder is selected from the group consisting of aberrant wound healing, cancer, and metastases, and
wherein said method comprises:
(a) contacting said sample with a nucleic acid probe which hybridizes under hybridization assay conditions to a nucleic acid target region of PTP LAR, said probe comprising the nucleic acid sequence encoding said PTP LAR, fragments thereof, or the complements of said sequences or fragments; and
(b) detecting the presence or amount of the probe:target region hybrid as an indication of said prognosis of said disease or disorder.

2. A method for prognosis of a disease or disorder by detection of PTP LAR in a sample,
wherein said sample is selected from the group consisting of cells, nucleic acid extracts of cells and biological fluids,
wherein said disease or disorder is selected from the group consisting of aberrant wound healing, cancer, and metastases, and
wherein said method comprises:
(a) comparing a nucleic acid target region encoding said PTP LAR in a sample with a control nucleic acid target region encoding said PTP LAR; and
(b) detecting differences in sequence or amount between said target region and said control target region, as an indication of said prognosis of said disease or disorder.

3. A method for detection of PTP LAR in a sample as a prognostic tool for a disease or disorder,
wherein said sample is selected from the group consisting of cells, proteins or membrane extracts of cells and biological fluids,
wherein said disease or disorder is selected from the group consisting of aberrant wound healing, cancer, and metastases, and
wherein said method comprises:
(a) contacting said sample with an antibody which hybridizes under hybridization assay conditions to a amino acid target region of PTP LAR,; and
(b) detecting the presence or amount of the antibody: target region complex as an indication of said prognosis of said disease or disorder.

4. The method of claim 3, wherein said antibody is selected from the group consisting of polyclonal and monoclonal antibodies.

5. The method of claim 3, wherein said antibody is from a hybridoma.

6. A method for prognosis of a disease or disorder by detection of PTP LAR in a sample,
wherein said sample is selected from the group consisting of cells, proteins or membrane extracts of cells and biological fluids,
wherein said disease or disorder is selected from the group consisting of aberrant wound healing, cancer, and metastases, and
wherein said method comprises detecting the presence or amount of PTP LAR as an indication of said prognosis of said disease or disorder.

7. A method for identifying one or more compounds that modulate eptithelial cell migration, comprising:
(a) contacting tyrosine phosphorylated β-catenin with one or more potential compounds;
(b) monitoring a change in the phosphorylation level of said tyrosine phosphorylated β-catenin to identify said one or more compounds that modulate said epithelial cell migration.

8. The method of claim 7, wherein said one or more compounds are selected from the group consisting of indolinones, quinazolines, quinoxalines, and tyrphostins.

9. A method for identifying one or more compounds that modulate PTP LAR activity, comprising:
(a) contacting PTP LAR with one or more potential compounds;
(b) measuring the activity of said PTP LAR; and
(c) comparing the activity of said PTP LAR to the activity of PTP LAR that has not been contacted with one or more potential compounds in order to determine if the compound is an agonist or antagonist of PTP LAR.

10. The method of claim 9, wherein said activity is phosphotyrisine phosphatase activity.

11. The method of claim 9, further comprising the addition of a natural binding partner to part (a).

12. The method of claim 11, wherein said natural binding partner is selected from the group consisting of β-catenin and plakoglobin.

13. The method of claim 9, wherein said one or more compounds are selected from the group consisting of indolinones, quinazolines, quinoxalines, and tyrphostins.

14. A method for identifying one or more compounds that modulate PTP LAR activity in cells, comprising:
(a) expressing PTP LAR activity in said cells;
(b) contacting said cells with one or more potential compounds; and
(c) monitoring a change in cell migration or the interaction between said PTP LAR and a natural binding partner to identify said one or more compounds that modulate said PTP LAR activity.

15. The method of claim 14, wherein said natural binding partner is selected from the group consisting of β-catenin, and plakoglobin.

16. The method of claim 14, wherein said one or more compounds are selected from the group consisting of indolinones, quinazolines, quinoxalines, and tyrphostins.

## Patentansprüche

1. Verfahren zur Prognose einer Krankheit oder Funktionsstörung durch Detektion von PTP LAR in einer Probe,
wobei die Probe aus der Gruppe ausgewählt wird, welche aus Zellen, Nukleinsäureextrakten von Zellen und biologischen Flüssigkeiten besteht,
wobei die Krankheit oder Funktionsstörung aus der Gruppe ausgewählt wird, welche aus aberranter Wundheilung, Krebs und Metastasen besteht, und
wobei das Verfahren umfasst:
(a) Inkontaktbringen der Probe mit einer Nukleinsäuresonde, die unter Bedingungen eines Hybridisierungsassays mit einer Nukleinsäurezielregion von PTP LAR hybridisiert, wobei die Sonde die Nukleinsäuresequenz umfasst, welche PTP LAR, Fragmente davon oder die Komplemente dieser Sequenzen oder Fragmente kodiert; und
(b) Detektieren der Gegenwart oder der Menge des Sonde/Targetregion-Hybrids als Indikation der Prognose der Krankheit oder Funktionsstörung.

2. Verfahren zur Prognose einer Krankheit oder Funktionsstörung durch Detektion von PTP LAR in einer Probe,
wobei die Probe aus der Gruppe ausgewählt wird, welche aus Zellen, Nukleinsäureextrakten von Zellen und biologischen Flüssigkeiten besteht,
wobei die Krankheit oder Funktionsstörung aus der Gruppe ausgewählt wird, welche aus aberranter Wundheilung, Krebs und Metastasen besteht, und
wobei das Verfahren umfasst:
(a) Vergleichen einer Nukleinsäurezielregion, welche die PTP LAR kodiert, in einer Probe mit einer Kontroll-Nukleinsäurezielregion, welche die PTP LAR kodiert; und
(b) Detektieren von Unterschieden in der Sequenz oder der Menge zwischen der Zielregion und der Kontroll-Zielregion als Indikation der Prognose der Krankheit oder Funktionsstörung.

3. Verfahren zur Detektion von PTP LAR in einer Probe als ein prognostisches Hilfsmittel für eine Krankheit oder Funktionsstörung,
wobei die Probe aus der Gruppe ausgewählt wird, welche aus Zellen, Proteinen oder Membranextrakten von Zellen und biologischen Flüssigkeiten besteht
wobei die Krankheit oder Funktionsstörung aus der Gruppe ausgewählt wird, welche aus aberranter Wundheilung, Krebs und Metastasen besteht, und
wobei das Verfahren umfasst:
(a) Inkontaktbringen der Probe mit einem Antikörper, der unter Bedingungen eines Hybridisierungsassays mit einer Aminosäurezielregion von PTP LAR hybridisiert; und
(b) Detektieren der Gegenwart oder der Menge des Antikörper/Targetregion-Komplexes als Indikation der Prognose der Krankheit oder Funktionsstörung.

4. Verfahren gemäß Anspruch 3, wobei der Antikörper aus der Gruppe ausgewählt wird, die aus polyklonalen und monoklonalen Antikörpern besteht.

5. Verfahren gemäß Anspruch 3, wobei der Antikörper aus einer Hybridomazelle ist.

6. Verfahren zur Prognose einer Krankheit oder Funktionsstörung durch Detektion von PTP LAR in einer Probe,
wobei die Probe aus der Gruppe ausgewählt wird, welche aus Zellen, Proteinen oder Membranextrakten von Zellen und biologischen Flüssigkeiten besteht
wobei die Krankheit oder Funktionsstörung aus der Gruppe ausgewählt wird, welche aus aberranter Wundheilung, Krebs und Metastasen besteht, und
wobei das Verfahren das Detektieren der Gegenwart oder Menge von PTP LAR als Indikation der Prognose der Krankheit oder Funktionsstörung umfasst.

7. Verfahren zur Identifikation einer oder mehr Verbindungen, welche die epitheliale Zellmigration modulieren, das umfasst:
(a) Inkontaktbringen von tyrosin-phosphoryliertem β-Catenin mit einer oder mehr potentiellen Verbindungen; und
(b) Überwachen einer Veränderung des Phosphorylierungsgrads von tyrosin-phosphoryliertem β-catenin, um eine oder mehr Verbindungen zu identifizieren, welche die epitheliale Zellmigration modulieren.

8. Verfahren gemäß Anspruch 7, wobei die eine oder mehr Verbindungen aus der Gruppe ausgewählt werden, die aus Indolinonen, Quinazolinen, Quinoxalinen und Tyrphostinen besteht.

9. Verfahren zur Identifikation von einer oder mehr Verbindungen, welche die Aktivität von PTP LAR modulieren, das umfasst:
(a) Inkontaktbringen von PTP LAR mit einer oder mehr potentiellen Verbindungen;
(b) Messen der Aktivität von PTP LAR; und
(c) Vergleichen der Aktivität von PTP LAR mit der Aktivität von PTP LAR, die nicht mit einer oder mehr potentieller Verbindungen in Kontakt gebracht wurde, um zu bestimmen, ob die Verbindung ein Agonist oder Antagonist von PTP LAR ist.

10. Verfahren gemäß Anspruch 9, wobei die Aktivität die Phosphotyrosin-Phosphataseaktivität ist.

11. Verfahren gemäß Anspruch 9, das ferner die Zugabe eines natürlichen Bindungspartners zu Schritt (a) umfasst.

12. Verfahren gemäß Anspruch 11, wobei der natürliche Bindungspartner aus der Gruppe ausgewählt wird, die aus β-Catenin und Plakoglobin besteht.

13. Verfahren gemäß Anspruch 9, wobei die eine oder mehr Verbindungen aus der Gruppe ausgewählt werden, die aus Indolinonen, Quinazolinen, Quinoxalinen und Tyrphostinen besteht.

14. Verfahren zur Identifikation von einer oder mehr Verbindungen, welche die Aktivität von PTP LAR in Zellen modulieren, das umfasst:
(a) Exprimieren der PTP LAR Aktivität in den Zellen;
(b) Inkontaktbringen der Zellen mit einer oder mehr potentiellen Verbindungen; und
(c) Überwachen einer Veränderung in der Zellmigration oder der Interaktion der PTP LAR und einem natürlichen Bindungspartner, um eine oder mehr Verbindungen zu identifizieren, welche die Aktivität von PTP LAR modulieren.

15. Verfahren gemäß Anspruch 14, wobei der natürliche Bindungspartner aus der Gruppe ausgewählt wird, die aus β-Catenin und Plakoglobin besteht.

16. Verfahren gemäß Anspruch 14, wobei die eine oder mehr Verbindungen aus der Gruppe ausgewählt werden, die aus Indolinonen, Quinazolinen, Quinoxalinen und Tyrphostinen besteht.

## Revendications

1. Procédé pour le pronostic d'une maladie ou d'une affection par détection de PTP LAR dans un échantillon,
ledit échantillon étant sélectionné dans le groupe consistant en cellules, extraits d'acides nucléiques de cellules et fluides biologiques,
ladite maladie ou affection étant sélectionnée dans le groupe consistant en cicatrisation aberrante, cancer et métastases, et
ledit procédé comprenant les étapes consistant à :
(a) mettre en contact ledit échantillon avec une sonde d'acides nucléiques qui s'hybride dans des conditions de test d'hybridation à une région d'acides nucléiques cible de PTP LAR, ladite sonde comprenant la séquence d'acide nucléique codant ledit PTP LAR, des fragments de celle-ci ou les compléments desdites séquences ou fragments ; et
(b) détecter la présence ou une quantité de l'hybride sonde:région cible, en tant qu'indication dudit pronostic de ladite maladie ou affection.

2. Procédé pour le pronostic d'une maladie ou d'une affection par détection de PTP LAR dans un échantillon,
ledit échantillon étant sélectionné dans le groupe consistant en cellules, extraits d'acide nucléique de cellules et fluides biologiques,
ladite maladie ou affection étant sélectionnée dans le groupe consistant en cicatrisation aberrante, cancer et métastases,
ledit procédé comprenant les étapes consistant à :
(a) le fait de comparer une région d'acides nucléiques cible codant ledit PTP LAR dans un échantillon avec une région d'acides nucléiques cible témoin codant ledit PTP LAR ; et
(b) détecter des différences de séquence ou de quantité entre ladite région cible et ladite région cible témoin, en tant qu'indication dudit pronostic de ladite maladie ou affection.

3. Procédé pour la détection de PTP LAR dans un échantillon en tant qu'outil pronostique pour une maladie ou une affection,
ledit échantillon étant sélectionné dans le groupe consistant en cellules, protéines ou extraits membranaires de cellules et fluides biologiques,
ladite maladie ou affection étant sélectionnée dans le groupe consistant en cicatrisation aberrante, cancer et métastases, et
ledit procédé comprenant les étapes consistant à :
(a) mettre en contact ledit échantillon avec un anticorps qui s'hybride dans des conditions de test d'hybridation à une région cible d'acides aminés de PTP LAR ; et
(b) détecter la présence ou une quantité de complexe anticorps:région cible, en tant qu'indication dudit pronostic de ladite maladie ou affection.

4. Procédé selon la revendication 3, dans lequel ledit anticorps est sélectionné dans le groupe consistant en anticorps polyclonaux et monoclonaux.

5. Procédé selon la revendication 3, dans lequel ledit anticorps provient d'un hybridome.

6. Procédé pour le pronostic d'une maladie ou d'une affection par détection de PTP LAR dans un échantillon,
ledit échantillon étant sélectionné dans le groupe consistant en cellules, protéines ou extraits membranaires de cellules et fluides biologiques,
ladite maladie ou affection étant sélectionnée dans le groupe consistant en cicatrisation aberrante, cancer et métastases,
ledit procédé comprenant le fait de détecter la présence ou la quantité de PTP LAR, en tant qu'indication dudit pronostic de ladite maladie ou affection.

7. Procédé pour identifier un ou plusieurs composés qui modulent la migration des cellules épithéliales, comprenant les étapes consistant à :
(a) mettre en contact de la β-caténine à résidus tyrosine phosphorylés avec un ou plusieurs composés potentiels ;
(b) le fait de suivre un changement du niveau de phosphorylation de ladite β-caténine à résidus tyrosine phosphorylés pour identifier un ou plusieurs composés qui modulent ladite migration des cellules épithéliales.

8. Procédé selon la revendication 7, dans lequel lesdits un ou plusieurs composés sont sélectionnés dans le groupe consistant en indolinones, quinazolines, quinoxalines, et tyrphostines.

9. Procédé pour identifier un ou plusieurs composés qui modulent l'activité PTP LAR, comprenant les étapes consistant à :
(a) mettre en contact PTP LAR avec un ou plusieurs composés potentiels ;
(b) mesurer l'activité dudit PTP LAR ; et
(c) comparer l'activité dudit PTP LAR à l'activité de PTP LAR qui n'a pas été mis en contact avec un ou plusieurs composés potentiels, afin de déterminer si le composé est un agoniste ou un antagoniste de PTP LAR.

10. Procédé selon la revendication 9, dans lequel ladite activité est une activité phosphotyrosine phosphatase.

11. Procédé selon la revendication 9, comprenant en outre l'addition d'un partenaire de liaison naturel à la partie (a).

12. Procédé selon la revendication 11, dans lequel ledit partenaire de liaison naturel est sélectionné dans le groupe consistant en β-caténine et plakoglobine.

13. Procédé selon la revendication 9, dans lequel lesdits un ou plusieurs composés sont sélectionnés dans le groupe consistant en indolinones, quinazolines, quinoxalines, et tyrphostines.

14. Procédé pour identifier un ou plusieurs composés qui modulent l'activité PTP LAR dans des cellules, comprenant les étapes consistant à :
(a) exprimer une activité PTP LAR dans lesdites cellules ;
(b) mettre en contact lesdites cellules avec un ou plusieurs composés potentiels ; et
(c) suivre un changement dans la migration des cellules ou dans l'interaction entre ledit PTP LAR et un partenaire de liaison naturel pour identifier lesdits un ou plusieurs composés qui modulent ladite activité PTP LAR.

15. Procédé selon la revendication 14, dans lequel ledit partenaire de liaison naturel est sélectionné dans le groupe consistant en β-caténine et plakoglobine.

16. Procédé selon la revendication 14, dans lequel lesdits un ou plusieurs composés sont sélectionnés dans le groupe consistant en indolinones, quinazolines, quinoxalines, et tyrphostines.
